# EUROPEAN PATENT APPLICATION

(11) **EP 4 722 254 A1**
(43) Date of publication of application: **08.04.2026**
(21) Application number: 24842445.9
(22) Date of filing: 19.07.2024
(51) Int. Cl.: C07K 16/46, C12N 15/13, A61K 39/395, A61P 35/00, A61K 47/68

(54) **ANTI-FGFR2/PD-1 BISPECIFIC ANTIBODY**

(30) Priority: 20.07.2023 CN 202310895582
(71) Applicant: Sunshine Guojian Pharmaceutical (Shanghai) Co., Ltd., Shanghai 201203 (CN)
(72) Inventor: HUANG, Haomin, Shanghai 201203 (CN); WANG, Dinghe, Shanghai 201203 (CN); LOU, Jing, Shanghai 201203 (CN)
(74) Representative: dompatent
(86) International application number: PCT/CN2024/106567
(87) International publication number: WO 2025/016459

(57) **Abstract**

The present invention relates to an anti-FGFR2/PD-1 bispecific antibody. The anti-FGFR2/PD-1 bispecific antibody of the present invention comprises a first antigen-binding domain D1 and a second antigen-binding domain D2, wherein D1 is an anti-FGFR2 antibody or an antigen-binding fragment thereof, and D2 is an anti-PD-1 antibody or an antigen-binding fragment thereof. The anti-FGFR2/PD-1 bispecific antibody of the present invention can inhibit the proliferation of FGFR2 target cells and kill the target cells by means of an ADCC effect, and can also block the binding of PD-1 to a ligand thereof, remove a T cell suppressive state and exert an anti-tumor effect.

## Description

### TECHNICAL FIELD

The present invention relates to the field of antibody drug technology, and specifically, relates to an anti-FGFR2/PD-1 bispecific antibody.

### BACKGROUND ART

Fibroblast growth factor receptors (FGFRs) are highly conserved receptor tyrosine kinases, commonly including four typical structures FGFR1-4, and one FGFR5 lacking the intracellular tyrosine kinase domain. The FGFR extracellular domain contains three immunoglobulin-like domains, wherein carboxyl-selective isomerization of the third Ig domain can produce the IIIb or IIIc subtypes of FGFR1 to FGFR3. FGFR2-IIIb expressed on epithelial cells can be activated by FGFs and forms a ternary complex with heparan sulfate proteoglycans, mediating signaling pathways such as RAS-RAF-MAPK, PI3K-AKT, signal transducers and activators of transcription, and phospholipase Cγ, participating in physiological processes such as angiogenesis, cell proliferation and migration, regulation of organ development, and wound healing. After abnormalities occur in FGFR2 signaling, including overexpression of FGFR2 and its ligands, mutations in the FGFR2 receptor, and switching of its subtypes, can induce normal cell carcinogenesis, such as FGFR2 single nucleotide polymorphisms in breast cancer cells, and overexpression of FGFR2-IIIb and its ligands in pancreatic cancer cells. Currently approved drugs targeting this target, such as erdafitinib from Johnson & Johnson, pemigatinib from Incyte, and infigratinib from BridgeBio Pharma, are all small molecule inhibitors. There are relatively few macromolecular antibody drugs under development, such as the bemarituzumab monoclonal antibody targeting FGFR2 for the treatment of HER2-negative locally advanced or metastatic gastric cancer and gastroesophageal junction cancer, which is still in the clinical development stage.

Programmed cell death protein 1 (PD-1), also known as CD279, belongs to the immunoglobulin superfamily, is a type I membrane protein composed of 288 amino acids, and is a member of the expanded CD28/CTLA-4 family of T cell regulators. Its structure includes an extracellular IgV domain, a transmembrane region, and an intracellular tail. The intracellular tail contains two phosphorylation sites for immunoreceptor tyrosine-based inhibitory and activating motifs, which can bind to SHP-1 and SHP-2 phosphatases. PD-1 is expressed on the surface of activated T cells, B cells, macrophages, etc. The interaction between the PD-1 protein expressed on the surface of activated immune cells and its ligands PD-L1 and PD-L2 upregulates the E3-ubiquitin ligases CBL-b and c-CBL, triggers downregulation of immune cell receptors, inhibits their activation and cytokine release, or completely initiates programmed death of immune cells. High expression of PD-L1 and PD-L2 has been found in various solid tumors and hematological malignancies, indicating that tumor cells utilize the immune suppression response mediated by PD-1 and its ligands to achieve immune escape. In 2018, the world's first PD-1 inhibitor, Nivolumab, was approved for marketing in China for the second-line treatment of advanced non-small cell lung cancer with wild-type EGFR/ALK driver genes. Subsequently, the first domestic PD-1 antibody drug, Sintilimab, for the treatment of Hodgkin's lymphoma, was also approved for marketing. With more in-depth clinical research on PD-1, PD-1 drugs have made major breakthroughs in the field of cancer treatment, becoming the most popular tumor immunotherapy method in recent years. Globally approved indications include melanoma, lymphoma, lung cancer, gastric cancer, liver cancer, colorectal cancer, etc.

Bispecific antibodies (BsAbs), also known as bifunctional antibodies, are specific drugs that simultaneously target two different antigens or different epitopes of the same antigen. BsAbs can direct immune cells and viral molecules to act on tumor cells, enhancing the killing effect on target cells. They can also simultaneously bind to different antigens on tumor cells, enhancing their binding specificity and reducing off-target effects. Bispecific antibodies broaden the application field of antibody drugs and provide new research ideas for tumor immunotherapy.

However, there is currently a lack of satisfactory bispecific antibodies targeting both FGFR2 and PD-1 in the art. Therefore, there is an urgent need in the art to develop a new bispecific antibody against FGFR2 and PD-1.

### SUMMARY OF THE INVENTION

The object of the present invention is to provide an anti-FGFR2/PD-1 bispecific antibody. The bispecific antibody provided by the present invention can simultaneously specifically bind to FGFR2 and PD-1, inhibit FGFR2 target cell proliferation and kill target cells through ADCC effects, while protecting T cells to normally exert immune functions and clear target cells. The object of the present invention is also to provide a polynucleotide molecule encoding said bispecific antibody; to provide an expression vector comprising said molecule; to provide a host cell comprising said expression vector; to provide a method for preparing said bispecific antibody; to provide a pharmaceutical composition comprising said bispecific antibody; to provide an immunoconjugate comprising said bispecific antibody; to provide the use of said bispecific antibody or said pharmaceutical composition in the manufacture of a drug for cancer or tumor; to provide a method for treating cancer or tumor using the fusion protein, the pharmaceutical composition or the immunoconjugate.

In a first aspect of the present invention, there is provided a bispecific antibody, comprising: a first antigen-binding domain D1; and
a second antigen-binding domain D2,
wherein, the D1 specifically binds to the target molecule FGFR2 protein,
the D2 specifically binds to the target molecule PD-1 protein.

In another preferred embodiment, D1 is an anti-FGFR2 antibody or an antigen-binding fragment thereof,

In another preferred embodiment, D2 is an anti-PD-1 antibody or an antigen-binding fragment thereof.

In another preferred embodiment, the antibody includes: animal-derived antibodies (such as murine antibodies), chimeric antibodies, humanized antibodies.

In another preferred embodiment, the antibody fragment comprises a heavy chain variable region and a light chain variable region.

In another preferred embodiment, the antibody fragment comprises a single-chain variable fragment (scFv), a double-chain variable fragment (dcFv).

In another preferred embodiment, D1 and D2 are connected via a linker.

In another preferred embodiment, D1 is an antibody, and the linker is (G4S)n, preferably, (G4S)n, wherein n is a positive integer (e.g., 1, 2, 3, 4, 5, or 6), preferably, n=3 or 4; more preferably, the linker is as shown in SEQ ID NO. 17 or SEQ ID NO. 18.

In another preferred embodiment, D1 is an anti-FGFR2 antibody, and D2 is connected via a linker to a region of D1 selected from the group consisting of: the heavy chain variable region, the heavy chain constant region, the light chain variable region, or a combination thereof.

In another preferred embodiment, D1 is an anti-FGFR2 antibody, and D2 is connected via a linker to the terminus of the heavy chain constant region of D1.

In another preferred embodiment, the bispecific antibody is a homodimer or heterodimer. In another preferred embodiment, the bispecific antibody comprises a monomer, having a structure represented by Formula I or Formula II from the N-terminus to the C-terminus: 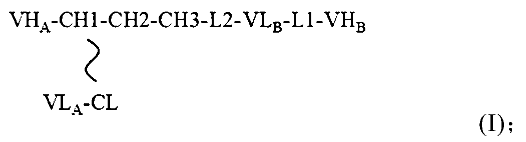 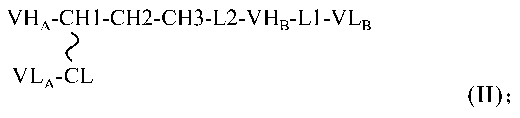 wherein,
VL_{A} represents the light chain variable region of the anti-FGFR2 antibody or antigen-binding fragment thereof;
VH_{A} represents the heavy chain variable region of the anti-FGFR2 antibody or antigen-binding fragment thereof;
VL_{B} represents the light chain variable region of the anti-PD-1 antibody or antigen-binding fragment thereof;
VH_{B} represents the heavy chain variable region of the anti-PD-1 antibody or antigen-binding fragment thereof;
CH represents the heavy chain constant region;
CL represents the light chain constant region;
L1 and L2 are each independently a bond or a linker;
"~" represents a disulfide bond or covalent bond;
"-" represents a peptide bond;
wherein, said bispecific antibody has the activity of simultaneously binding FGFR2 and binding PD-1.

In another preferred embodiment, the anti-FGFR2 or anti-PD-1 antigen-binding fragment is selected from the group consisting of Fab, F(ab'), F(ab')₂, Fv or single-chain Fv (scFv); the anti-FGFR2 or anti-PD-1 antibody is an IgG antibody.

In another preferred embodiment, the anti-FGFR2 or anti-PD-1 antibody is selected from the group consisting of a chimeric antibody, a murine antibody, or a humanized antibody. In another preferred embodiment, the IgG antibody comprises a heavy chain constant region and a light chain constant region; more preferably, the heavy chain constant region is selected from the group consisting of human IgG1, human IgG2, human IgG3, or human IgG4, and the light chain constant region is selected from the group consisting of human κ (Kappa) or human λ (Lambda).

In another preferred embodiment, the constant region of the IgG antibody comprises at least one amino acid mutation.

In another preferred embodiment, the D1 is an IgG antibody, and the D2 is an scFv; more preferably, the D2 is connected to the N-terminus or C-terminus of D1; even more preferably, the D2 is connected to the heavy chain of D1.

In another preferred embodiment, the D2 comprises one, two, three, or more anti-PD-1 scFvs.

In another preferred embodiment, the scFv comprises, from N-terminus to C-terminus, a VH-L1-VL structure or a VL-L1-VH structure.

In another preferred embodiment, the amino acid sequences of the linkers L1 and L2 are independently (G4S)ₙ, wherein n is selected from 1, 2, 3, 4, 5, 6.

In another preferred embodiment, the amino acid sequence of the linker L1 is as shown in SEQ ID NO. 17.

In another preferred embodiment, the amino acid sequence of the linker L2 is as shown in SEQ ID NO. 18.

In another preferred embodiment, the bispecific antibody is a homodimer, comprising an anti-FGFR2 IgG antibody and two anti-PD-1 scFvs, wherein each scFv comprises a variable region VH and a variable region VL, VH and VL are connected via linker L1 to form a VL-L1-VH structure, and each anti-PD-1 scFv is connected to the C-terminus of the anti-FGFR2 immunoglobulin antibody IgG via linker L2.

In another preferred embodiment, the bispecific antibody comprises a heavy chain and a light chain, and the amino acid sequences of the heavy chain and light chain are selected from the group consisting of:
a) the amino acid sequence of the heavy chain of the bispecific antibody is as shown in SEQ ID NO. 29, and the amino acid sequence of the light chain of the bispecific antibody is as shown in SEQ ID NO. 28; and
b) the amino acid sequence of the heavy chain of the bispecific antibody is as shown in SEQ ID NO. 30, and the amino acid sequence of the light chain of the bispecific antibody is as shown in SEQ ID NO. 28; or,
c) a polypeptide derived from a) or b) formed by substitution, deletion, or addition of one or more amino acid residues and having both anti-FGFR2 activity and anti-PD-1 activity.

In another preferred embodiment, the bispecific antibody includes active fragments and/or derivatives of said bispecific antibody, wherein the active fragments and/or the derivatives retain 70-100% (such as 70%, 75%, 80%, 85%, 90%, 95%, 96%, 97%, 98%, 99%, 100%) of the anti-FGFR2 activity and 70-100% of the anti-PD-1 activity of said bispecific antibody. In another preferred embodiment, the derivative of the bispecific antibody is a polypeptide derived from the bispecific antibody after one or several amino acid mutations (amino acid deletion, insertion, and/or substitution) and maintaining at least 85%, 86%, 87%, 88%, 89%, 90%, 91%, 92%, 93%, 94%, 95%, 96%, 97%, 98%, 99% sequence identity.

In another preferred embodiment, the amino acid mutation is a conservative amino acid substitution.

In another preferred embodiment, the amino acid mutation is located in the framework region or constant region.

In another preferred embodiment, the anti-FGFR2 antibody or antigen-binding fragment thereof comprises a heavy chain variable region and a light chain variable region, the heavy chain variable region comprising the following three heavy chain complementarity determining regions: HCDR1 as shown in SEQ ID NO. 1, HCDR2 as shown in SEQ ID NO. 2, HCDR3 as shown in SEQ ID NO. 3; and
the light chain variable region comprises the following three light chain complementarity determining regions: LCDR1 as shown in SEQ ID NO. 4, LCDR2 as shown in SEQ ID NO. 5, LCDR3 as shown in SEQ ID NO. 6.

In another preferred embodiment, the anti-PD-1 antibody or antigen-binding fragment thereof comprises a heavy chain variable region and a light chain variable region, the heavy chain variable region comprising the following three heavy chain complementarity determining regions: HCDR1 as shown in SEQ ID NO. 7, HCDR2 as shown in SEQ ID NO. 8, HCDR3 as shown in SEQ ID NO. 9; and
the light chain variable region comprises the following three light chain complementarity determining regions: LCDR1 as shown in SEQ ID NO. 10, LCDR2 as shown in SEQ ID NO. 11, LCDR3 as shown in SEQ ID NO. 12.

In another preferred embodiment, the anti-FGFR2 antibody or antigen-binding fragment thereof comprises a heavy chain variable region and a light chain variable region, the amino acid sequence of the heavy chain variable region is as shown in SEQ ID NO. 13, and the amino acid sequence of said light chain variable region is as shown in SEQ ID NO. 14.

In another preferred embodiment, the anti-PD-1 antibody or antigen-binding fragment thereof comprises a heavy chain variable region and a light chain variable region, the amino acid sequence of the heavy chain variable region is as shown in SEQ ID NO. 15, and the amino acid sequence of the light chain variable region is as shown in SEQ ID NO. 16.

In another preferred embodiment, the heavy chain constant region comprises an Fc segment, and said Fc segment is derived from IgG1 or IgG4.

In another preferred embodiment, the Fc segment is an Fc segment derived from IgG1.

In another preferred embodiment, the Fc segment comprises at least one amino acid mutation.

In another preferred embodiment, the mutation is used to enhance the ADCC effect of the bispecific antibody.

In another preferred embodiment, the Fc segment derived from IgG1 has mutations selected from the group consisting of:
S239D, I332E, A330L, G236A, preferably S239D, I332E.

In another preferred embodiment, the heavy chain constant region comprises CH1, CH2, CH3.

In another preferred embodiment, the amino acid sequences of the CH1, CH2, CH3 are independently as shown in SEQ ID NO. 19, SEQ ID NO. 20, SEQ ID NO. 21.

In another preferred embodiment, the amino acid sequence of the heavy chain constant region is as shown in SEQ ID NO. 23.

In another preferred embodiment, the heavy chain constant region comprises amino acid mutations selected from the group consisting of: S239D, I332E.

In another preferred embodiment, the anti-FGFR2 antibody or antigen-binding fragment thereof comprises a heavy chain variable region and a light chain variable region, wherein the amino acid sequence of the heavy chain variable region is as shown in SEQ ID NO. 13, and the amino acid sequence of the light chain variable region is as shown in SEQ ID NO. 14; and/or, the anti-PD1 antibody or antigen-binding fragment thereof comprises a heavy chain variable region and a light chain variable region, wherein the amino acid sequence of the heavy chain variable region is as shown in SEQ ID NO. 15, and the amino acid sequence of the light chain variable region is as shown in SEQ ID NO. 16;
preferably, the variable region comprises at least one amino acid mutation.

In another preferred embodiment, the heavy chain constant region of the anti-FGFR antibody comprises amino acid mutations selected from the group consisting of:
a) the heavy chain constant region has an amino acid mutation at position 239; preferably S293D; and/or
b) the heavy chain constant region has an amino acid mutation at position 332; preferably I332E.

In another preferred embodiment, the heavy chain constant region sequence of the anti-FGFR antibody is as shown in SEQ ID NO. 23; or the heavy chain constant region sequence of the anti-FGFR antibody is as shown in SEQ ID NO. 24.

In another preferred embodiment, the anti-FGFR or anti-PD1 antigen-binding fragment is selected from the group consisting of Fab, F(ab'), F(ab')2, Fv or single-chain Fv (scFv); the anti-FGFR or anti-PD1 antibody is an IgG antibody.

In another preferred embodiment, D1 is an IgG antibody, and D2 is an scFv; preferably, D2 is connected to the N-terminus or C-terminus of D1; more preferably, D2 is connected to the heavy chain of D1.

In a second aspect of the present invention, there is provided a polynucleotide molecule encoding the bispecific antibody provided in the first aspect of the present invention.

In a third aspect of the present invention, there is provided an expression vector comprising the polynucleotide molecule according to the second aspect of the present invention.

In a fourth aspect of the present invention, there is provided a host cell comprising the expression vector according to the third aspect of the present invention.

In a fifth aspect of the present invention, there is provided a method for preparing the bispecific antibody provided in the first aspect of the present invention, the preparation method comprising the following steps:
a) culturing the host cell according to the fourth aspect of the present invention under expression conditions, thereby expressing the bispecific antibody;
b) isolating and purifying the bispecific antibody from step a).

In a sixth aspect of the present invention, there is provided a pharmaceutical composition comprising an effective amount of the bispecific antibody according to the first aspect of the present invention and one or more pharmaceutically acceptable carriers, diluents, or excipients.

In a seventh aspect of the present invention, there is provided a use of the bispecific antibody provided in the first aspect of the present invention or the pharmaceutical composition according to the sixth aspect of the present invention in the manufacture of a drug for cancer or tumor;
preferably, the cancer or tumor is an FGFR2-positive cancer or tumor.

In another preferred embodiment, the cancer or tumor is selected from the group consisting of: lung cancer, bone cancer, gastric cancer, pancreatic cancer, prostate cancer, skin cancer, head and neck cancer, uterine cancer, ovarian cancer, testicular cancer, fallopian tube cancer, endometrial cancer, cervical cancer, vaginal cancer, vulvar cancer, rectal cancer, colon cancer, anal region cancer, breast cancer, esophageal cancer, small intestine cancer, endocrine system cancer, thyroid cancer, parathyroid cancer, adrenal cancer, urethral cancer, penile cancer, prostate cancer, pancreatic cancer, brain cancer, testicular cancer, lymphoma, transitional cell carcinoma, bladder cancer, kidney cancer, ureteral cancer, renal cell carcinoma, renal pelvis cancer, Hodgkin's lymphoma, non-Hodgkin's lymphoma, soft tissue sarcoma, pediatric solid tumor, lymphocytic lymphoma, central nervous system tumor, primary central nervous system lymphoma, spinal tumor, brainstem glioma, pituitary adenoma, melanoma, Kaposi's sarcoma, epidermoid carcinoma, squamous cell carcinoma, T-cell lymphoma, chronic or acute leukemia, or a combination thereof.

In an eighth aspect of the present invention, there is provided an immunoconjugate comprising:
a) the bispecific antibody according to any one of the first aspect of the present invention; and
b) a conjugate moiety selected from the group consisting of: a detectable label, a drug, a toxin, a cytokine, a radionuclide, or an enzyme.

In another preferred embodiment, the conjugate moiety is selected from the group consisting of: a fluorescent or luminescent label, a radioactive label, a contrast agent for magnetic resonance imaging or computed tomography, or an enzyme capable of producing a detectable product, a radionuclide, a biotoxin, or a cytokine.

In another preferred embodiment, the immunoconjugate comprises an antibody-drug conjugate.

In another preferred embodiment, the immunoconjugate is used for preparing a pharmaceutical composition for treating tumor or cancer.

In a ninth aspect of the present invention, there is provided a method for treating cancer or tumor, the method comprising administering to a subject in need the bispecific antibody according to the first aspect of the present invention, the pharmaceutical composition according to the sixth aspect of the present invention, or the immunoconjugate according to the eighth aspect of the present invention.

In another preferred embodiment, the method further comprises co-administration with other anti-tumor drugs.

It should be understood that each of the above technical features of the present invention and each of the technical features specifically described below (e.g., in the Examples) can be combined with each other within the scope of the present invention to thus form new or preferred technical solutions, which will not be repeated here one by one due to space limitation.

### BRIEF DESCRIPTION OF DRAWINGS

FIG. 1A shows a schematic structural diagram of anti-FGFR2/PD-1 bispecific antibody a or b.
FIG. 1B shows a schematic structural diagram of anti-FGFR2/PD-1 bispecific antibody c.
FIG. 1C shows a schematic structural diagram of anti-FGFR2/PD-1 bispecific antibody d.
FIG. 2A shows the UPLC-SEC detection profile of anti-FGFR2/PD-1 bispecific antibody a.
FIG. 2B shows the UPLC-SEC detection profile of anti-FGFR2/PD-1 bispecific antibody b.
FIG. 2C shows the UPLC-SEC detection profile of anti-FGFR2/PD-1 bispecific antibody c.
FIG. 2D shows the UPLC-SEC detection profile of anti-FGFR2/PD-1 bispecific antibody d.
FIG. 3A shows the deN-glycosylated intact mass spectrum of anti-FGFR2/PD-1 bispecific antibody a.
FIG. 3B shows the deN-glycosylated intact mass spectrum of anti-FGFR2/PD-1 bispecific antibody b.
FIG. 4A shows the DSC detection profile of anti-FGFR2/PD-1 bispecific antibody a.
FIG. 4B shows the DSC detection profile of anti-FGFR2/PD-1 bispecific antibody b.
FIG. 5A shows ELISA detection of the binding of anti-FGFR2/PD-1 bispecific antibodies a, b to FGFR2.
FIG. 5B shows ELISA detection of the binding of anti-FGFR2/PD-1 bispecific antibodies c, d to FGFR2.
FIG. 5C shows ELISA detection of the binding of anti-FGFR2/PD-1 bispecific antibodies a, b to PD-1.
FIG. 5D shows ELISA detection of the binding of anti-FGFR2/PD-1 bispecific antibodies a, d to PD-1.
FIG. 6A shows the binding activity of anti-FGFR2/PD-1 bispecific antibodies a, b to Jurkat-PD-1-NFAT-luc cells.
FIG. 6B shows the activity of anti-FGFR2/PD-1 bispecific antibodies a, b in blocking PD-1/PD-L1 binding.
FIG. 7A shows the ADCC effect of anti-FGFR2/PD-1 bispecific antibodies a, b on SNU-16 cells.
FIG. 7B shows the ADCC effect of anti-FGFR2/PD-1 bispecific antibodies a, b on T cells.
FIG. 8 shows the inhibitory effect of anti-FGFR2/PD-1 bispecific antibodies a, b on FGF1-induced Ba/F3-FGFR2IIIb cell proliferation.
FIG. 9A shows the anti-tumor effect of anti-FGFR2/PD-1 bispecific antibody b in the MC38 xenograft model.
FIG. 9B shows the toxic side effects of anti-FGFR2/PD-1 bispecific antibody b on mice in the MC38 xenograft model.
FIG. 10A shows the anti-tumor effect of anti-FGFR2/PD-1 bispecific antibodies a, b in the SNU-16 xenograft model.
FIG. 10B shows the toxic side effects of anti-FGFR2/PD-1 bispecific antibodies a, b on mice in the SNU-16 xenograft model.

### DETAILED DESCRIPTION OF THE PREFERRED EMBODIMENTS

Through extensive and in-depth research, the inventors have developed a novel bispecific antibody that targets both the tumor cell surface molecule FGFR2 and the immune cell surface molecule PD-1. This antibody retains the activity of both parental arms and is capable of simultaneously binding to the FGFR2 and PD-1 antigens. The bispecific antibody of the present invention can be used to achieve T cell-mediated immune responses while killing FGFR2-expressing tumor cells through ADCC effects. Therefore, the bispecific antibody of the present invention can be developed as an anti-tumor drug with superior efficacy. The present invention has been completed on this basis.

### Terms

To facilitate understanding of the present disclosure, certain terms are defined first. As used in this application, unless otherwise expressly provided herein, each of the following terms shall have the meaning given below.

The term "about" may refer to a value or set that is within an acceptable error range for a particular value or set as determined by those of ordinary skill in the art, and the value or set partially depends on how the value or set is measured or determined.

In the present invention, the terms "antibody (Ab)" and "immunoglobulin G (IgG)" are heterotetrameric glycoproteins having the same structural characteristics, consisting of two identical light chains (L) and two identical heavy chains (H). Each light chain is linked to a heavy chain by a covalent disulfide bond, while the number of disulfide bonds between heavy chains varies among different immunoglobulin isotypes. Each heavy chain and light chain also has regularly spaced intrachain disulfide bonds. Each heavy chain has a variable region (VH) at one end, followed by a constant region. The heavy chain constant region consists of three domains: CH1, CH2, and CH3. Each light chain has a variable region (VL) at one end and a constant region at the other end. The light chain constant region includes one domain, CL; the constant region of the light chain pairs with the CH1 domain of the heavy chain constant region, and the variable region of the light chain pairs with the variable region of the heavy chain. The constant regions do not directly participate in the binding of the antibody to the antigen, but they exhibit different effector functions, such as involvement in antibody-dependent cell-mediated cytotoxicity (ADCC). The heavy chain constant regions include IgG1, IgG2, IgG3, and IgG4 subtypes; the light chain constant regions include κ (Kappa) or λ (Lambda). The heavy and light chains of the antibody are covalently linked together by disulfide bonds between the CH1 domain of the heavy chain and the CL domain of the light chain, and the two heavy chains of the antibody are covalently linked together by interchain disulfide bonds formed in the hinge region.

The "immunoglobulin antibody IgG" described in the present invention is a molecule of about 150 kDa, composed of four peptide chains, containing two identical γ heavy chains of about 50 kDa and two identical light chains of about 25 kDa, thus having a tetrameric quaternary structure. The two heavy chains are connected to each other by disulfide bonds and each is connected to one light chain. The resulting tetramer has two identical halves, forming a fork-like or Y-shaped structure, with each end of the fork containing an identical antigen-binding site. IgG antibodies can be divided into several subclasses (e.g., IgG1, 2, 3, 4) based on minor differences in the amino acid sequences of the constant regions of the heavy chains.

In the present invention, the term "bispecific antibody (or bispecific Ab)" refers to an antibody molecule capable of simultaneously specifically binding to two antigens (targets) or two epitopes. Based on symmetry, bispecific antibodies can be divided into structurally symmetric and asymmetric molecules. Based on the number of binding sites, bispecific antibodies can be divided into bivalent, trivalent, tetravalent, and multivalent molecules. In the present invention, the term "monoclonal antibody (mAb)" refers to an antibody obtained from a substantially homogeneous population, i.e., the individual antibodies contained in the population are identical except for possibly a few naturally occurring mutations. Monoclonal antibodies are highly specific for a single antigenic site. Moreover, unlike conventional polyclonal antibody preparations (which typically include different antibodies directed against different epitopes), each monoclonal antibody is directed against a single epitope on the antigen. In addition to their specificity, the advantage of monoclonal antibodies is that they can be synthesized by hybridoma culture without being contaminated by other immunoglobulins. The modifier "monoclonal" indicates the character of the antibody as being obtained from a substantially homogeneous population of antibodies and should not be construed as requiring production of the antibody by any particular method. As used herein, the term "antigen-binding fragment" refers to a Fab fragment, Fab' fragment, F(ab')₂ fragment, single Fv fragment, etc., having antigen-binding activity. Non-limiting examples of antigen-binding fragments include: (i) Fab fragments; (ii) F(ab')₂ fragments; (iii) Fv fragments; or (iv) single-chain Fv (scFv). As used herein, the expression "antigen-binding fragment" also encompasses other engineered molecules internally, such as domain-specific antibodies, single-domain antibodies, domain-deleted antibodies, chimeric antibodies, CDR-grafted antibodies, diabodies, triabodies, tetrabodies, minibodies, nanobodies (e.g., monovalent nanobodies and bivalent nanobodies, etc.), small modular immunopharmaceuticals, and shark variable IgNAR domains, etc.

In the present invention, the terms "Fab" and "Fc" refer to the fact that papain can cleave an antibody into two identical Fab segments and one Fc segment. The Fab segment consists of the VH and CH1 of the antibody's heavy chain and the VL and CL domains of the light chain. The Fc segment, i.e., the fragment crystallizable (Fc), consists of the CH2 and CH3 domains of the antibody. The Fc segment has no antigen-binding activity and is the site where the antibody interacts with effector molecules or cells. The term "F(ab')₂" fragment antibody is obtained by digesting the entire IgG antibody with pepsin, removing most of the Fc region while keeping some hinge region intact, resulting in two antigen-binding F(ab') parts linked together by disulfide bonds.

In the present invention, the term "scFv" or "single-chain variable fragment scFv" refers to a single-chain antibody fragment (scFv), which is a fusion protein comprising the immunoglobulin heavy chain VH and light chain VL variable regions, where VH and VL are connected by a linker containing 15-25 amino acids, and said fusion protein retains the same antigen specificity as the intact immunoglobulin.

In the present invention, the term "Fv fragment" or "Fv antibody" refers to the smallest antibody fragment that contains the heavy chain variable region and light chain variable region of an antibody, but no constant region, and possesses all antigen-binding sites. In some embodiments, the Fv fragment may further comprise a polypeptide linker between the VH and VL domains, which facilitates the formation of the structure required for antigen binding.

In the present invention, the term "variable" indicates that certain parts of the variable regions of antibodies differ in sequence, forming the binding and specificity of various specific antibodies for their specific antigens. However, variability is not uniformly distributed throughout the antibody variable regions. It is concentrated in three segments called complementarity-determining regions (CDRs) or hypervariable regions in the heavy chain variable region and light chain variable region. The more conserved parts of the variable regions are called framework regions (FR). The natural variable regions of heavy and light chains each contain four FR regions, which are roughly in a β-sheet configuration, connected by three CDRs forming loop connections, and in some cases may form part of the β-sheet structure. The CDRs in each chain are held closely together by the FR regions and, together with the CDRs of the other chain, form the antigen-binding site of the antibody (see Kabat et al., NIH Publ. No. 91-3242, Vol. I, pages 647-669 (1991)).

As used herein, the term "framework region (FR)" refers to the amino acid sequences interposed between CDRs, i.e., those portions of the light and heavy chain variable regions of an immunoglobulin that are relatively conserved among different immunoglobulins in a single species. The light and heavy chains of an immunoglobulin each have four FRs, designated as FR1-L, FR2-L, FR3-L, FR4-L and FR1-H, FR2-H, FR3-H, FR4-H, respectively. Accordingly, the light chain variable domain can thus be referred to as (FR1-L)-(CDR1-L)-(FR2-L)-(CDR2-L)-(FR3-L)-(CDR3-L)-(FR4-L) and the heavy chain variable domain can be represented as (FR1-H)-(CDR1-H)-(FR2-H)-(CDR2-H)-(FR3-H)-(CDR3-H)-(FR4-H). Preferably, the FRs of the present invention are human antibody FRs or derivatives thereof, said derivatives of human antibody FRs being substantially identical to naturally occurring human antibody FRs, i.e., having sequence identity of 85%, 90%, 95%, 96%, 97%, 98%, or 99%. Once the amino acid sequence of the CDR is known, those skilled in the art can readily determine the framework regions FR1-L, FR2-L, FR3-L, FR4-L and/or FR1-H, FR2-H, FR3-H, FR4-H. As used herein, the term "human framework region" is a framework region that is substantially identical (about 85% or more, specifically 90%, 95%, 97%, 99%, or 100%) to the framework region of a naturally occurring human antibody.

As used herein, the term "linker" refers to one or more amino acid residues that are inserted into the immunoglobulin structural domains to provide sufficient mobility to the light and heavy chain domains for pleating of the domains into an exchanged dual variable region immunoglobulin. In the present invention, preferred linkers are L1 and L2, wherein L1 connects the VH and VL of a single-chain antibody (scFv), and L2 connects the scFv either between the CH1 and CH2 domains of the antibody heavy chain, or directly to the antibody heavy chain (e.g., to the N-terminus or C-terminus of the antibody heavy chain). Examples of suitable linkers include glycine (Gly) or serine (Ser) residues, and the identity and sequence of amino acid residues in the linker can vary depending on the type of secondary structural element needed to be achieved in the linker.

In the present invention, the terms "anti-", "bind", "specifically bind" refer to a non-random binding reaction between two molecules, such as the reaction between an antibody and the antigen it targets. Typically, an antibody binds to the antigen with an equilibrium dissociation constant (KD) of less than about 10⁻⁷ M, for example less than about 10⁻⁸ M, 10⁻⁹ M, 10⁻¹⁰ M, 10⁻¹¹ M, or smaller. In the present invention, the term "KD" refers to the equilibrium dissociation constant for a specific antibody-antigen interaction, which is used to describe the binding affinity between the antibody and the antigen. The smaller the equilibrium dissociation constant, the tighter the antibody-antigen binding, and the higher the affinity between the antibody and the antigen. For example, the binding affinity of an antibody to an antigen is measured using surface plasmon resonance (SPR) in a BIACORE instrument or the relative affinity of antibody binding to an antigen is measured using ELISA.

In the present invention, the term "epitope" refers to a polypeptide determinant that specifically binds to an antibody or the region in an antigen that is bound by an antibody. As used herein, "amino acid mutations in the heavy chain constant region" refer to amino acid mutations at corresponding positions in naturally occurring human IgG1, for example, amino acid mutations at position 239 or 332 in human IgG1.

As used herein, the bispecific antibody of the present invention also includes conservative variants thereof, which refer to polypeptides formed by replacing up to 10, preferably up to 8, more preferably up to 5, and most preferably up to 3 amino acids with amino acids having similar or related properties compared to the amino acid sequence of the antibody of the present invention. These conserved variant polypeptides are preferably produced by amino acid substitution according to Table A.

**Table A**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Bispecific Antibody

The bispecific antibody of the present invention is a bispecific antibody capable of specifically binding to FGFR2 and PD-1, comprising an anti-FGFR2 antibody portion and an anti-PD-1 antibody portion.

The bispecific antibody of the present invention can be a dimer, trimer, or multimer, preferably a homodimer or heterodimer. The anti-FGFR2 or anti-PD-1 antibody portion in the bispecific antibody of the present invention may include one or more antibodies or antigen-binding fragments thereof, preferably 1, 2, 3, 4, 5, or 6. The sequences of the present invention adopt the Kabat numbering system.

When constructing the bispecific antibody of the present invention, issues related to the chemical and physical stability of the bispecific antibody have also been addressed, such as expressing physically stable molecules, increasing thermal and salt-dependent stability, reducing aggregation, increasing solubility at high concentrations, and maintaining affinity for the two antigens FGFR2 and PD-1, respectively.

### Encoding Nucleic Acids and Expression Vectors

Another aspect of the present invention provides a polynucleotide molecule encoding the bispecific antibody described above. The polynucleotide of the present invention may be in a DNA or RNA form. The DNA form includes cDNA, genomic DNA or synthetic DNA. The DNA may be single-stranded or double-stranded. The DNA may be a coding or noncoding strand. Once the related sequence has been obtained, the recombination method may be used to obtain the sequence on a large scale. This is typically done by cloning into a vector, transferring into a cell, and then isolating the related sequence from proliferated host cells by a conventional method.

The preparation method of the polynucleotide molecule of the present invention is a conventional preparation method in the art, preferably including the following preparation methods: obtaining the polynucleotide molecule encoding the above monoclonal antibody through gene cloning techniques such as PCR, or obtaining the polynucleotide molecule encoding the above monoclonal antibody by full-sequence artificial synthesis.

Those skilled in the art know that nucleotide sequences encoding the amino acid sequences of the above bispecific antibody can be appropriately substituted, deleted, altered, inserted, or added to provide a homolog of the polynucleotide. Homologs of the polynucleotide in the present invention can be prepared by substituting, deleting, or adding one or more bases of the gene encoding the bispecific antibody within the range of maintaining antibody activity.

Another aspect of the present invention provides an expression vector comprising the above polynucleotide molecule.

These vectors can be used for transformation into appropriate host cells to enable them to express the protein. Wherein said expression vector is a conventional expression vector in the art, referring to an expression vector comprising appropriate regulatory sequences, such as promoter sequences, terminator sequences, polyadenylation sequences, enhancer sequences, marker genes and/or sequences, and other appropriate sequences. The expression vector may be a virus or plasmid, such as an appropriate phage or phagemid. For further technical details, please refer to, for example, Molecular Cloning: A Laboratory Manual, Second Edition, Cold Spring Harbor Laboratory Press, 1989, edited by Sambrook et al. Many known techniques and protocols for nucleic acid manipulation can be found in, for example, Current Protocols in Molecular Biology, Second Edition, edited by Ausubel et al. The expression vector of the present invention is preferably pDR1, pcDNA3.1(+), pcDNA3.4, pcDNA3.1/ZEO(+), pDHFR, pTT5, pDHFF, pGM-CSF, or pCHO 1.0, more preferably pcDNA3.4.

The present invention further provides a host cell comprising the above expression vector. The host cell of the present invention is a conventional host cell in the art, as long as it can stably self-replicate the above recombinant expression vector and effectively express the carried nucleotide. The host cell includes prokaryotic expression cells and eukaryotic expression cells, and is preferably selected from: COS, CHO (Chinese Hamster Ovary), NS0, sf9, sf21, DH5α, BL21(DE3), or TG1, more preferably *E.coli* TG1, BL21(DE3) cells (for expressing single-chain antibodies or Fab antibodies) or CHO-K1 cells (for expressing full-length IgG antibodies). Transforming the aforementioned expression vector into a host cell yields the preferred recombinant expression transformant of the present invention. The transformation method is a conventional transformation method in the art, preferably chemical transformation, heat shock, or electroporation.

### Preparation method

The culture method for the host cell of the present invention and the separation and purification method for the antibody are conventional methods in the art. For specific operational methods, please refer to the corresponding cell culture technical manuals and antibody separation and purification technical manuals. The preparation method for the anti-FGFR2/PD-1 bispecific antibody disclosed in the present invention comprises: culturing the above host cell under expression conditions to express the bispecific antibody capable of specifically binding to FGFR2 and PD-1; and separating and purifying the anti-FGFR2/PD-1 bispecific antibody. Using the above method, the recombinant protein can be purified into a substantially homogeneous substance.

The anti-FGFR2/PD-1 bispecific antibody disclosed in the present invention can be separated and purified using affinity chromatography. Depending on the characteristics of the affinity column used, conventional methods such as high-salt buffer or pH change can be employed to elute the anti-FGFR2/PD-1 bispecific antibody bound to the affinity column. The inventors of the present invention conducted detection experiments on the obtained anti-FGFR2/PD-1 bispecific antibody. The experimental results showed that the anti-FGFR2/PD-1 bispecific antibody binds well to target cells and antigens, exhibiting high affinity.

### Pharmaceutical composition

Another aspect of the present invention provides a composition, preferably, the composition is a pharmaceutical composition.

In the present invention, the term "pharmaceutical composition" means that the bispecific antibody of the present invention can be combined with a pharmaceutically acceptable carrier to form a pharmaceutical formulation composition to more stably exert therapeutic effects. These formulations can ensure the conformational integrity of the amino acid core sequence of the bispecific antibody disclosed in the present invention, while also protecting the multifunctional groups of the protein from degradation (including but not limited to aggregation, deamidation, or oxidation).

Typically, these substances may be formulated in a non-toxic, inert, and pharmaceutically acceptable aqueous carrier medium, with the pH typically of about 5.0-8.0, and preferably about 6.0-8.0, although the pH may vary depending on the nature of the substance to be formulated and the condition to be treated. The formulated pharmaceutical composition may be administered via conventional routes, including but not limited to: intravenous injection, intravenous infusion, subcutaneous injection, local injection, intramuscular injection, intratumoral injection, intraperitoneal injection (e.g., intraperitoneally), intracranial injection, or intracavitary injection. Typically, for liquid formulations, stability can be maintained for at least one year under conditions of 2-8°C; for lyophilized formulations, stability can be maintained for at least six months under conditions of 30°C. The bispecific antibody formulation can be a suspension, injection, lyophilized formulation, or other formulations commonly used in the pharmaceutical field.

The pharmaceutical composition of the present invention comprises a safe and effective amount (e.g., 0.001 wt% to 99 wt%, preferably 0.01 wt% to 90 wt%, more preferably 0.1 wt% to 80 wt%) of the bispecific antibody (or its conjugate) of the present invention as described above, and a pharmaceutically acceptable carrier or excipient. Such carriers include (but are not limited to): saline, buffer, glucose, water, glycerol, ethanol, and combinations thereof. The pharmaceutical formulation should match the route of administration. The pharmaceutical composition of the present invention may be prepared into an injectable form, for example, by conventional methods using normal saline or aqueous solutions containing glucose and other excipients. The pharmaceutical composition in a form such as an injection or a solution should be manufactured under sterile conditions. The administration amount of the active ingredient is a therapeutically effective amount, for example, about 10 micrograms/kg body weight to 50 milligrams/kg body weight per day. Furthermore, the bispecific antibody of the present invention can also be used in combination with other therapeutic agents.

When using the pharmaceutical composition, a safe and effective amount of the bispecific antibody or its immunoconjugate is administered to a mammal. Wherein the safe and effective amount is generally at least about 10 µg/kg body weight, and in most cases does not exceed about 50 mg/kg body weight; preferably, the dosage is about 10 µg/kg body weight to 10 mg/kg body weight. Without doubt, the specific dose should also take factors such as the route of administration and the patient's health status into consideration, all of which fall within the area of expertise of skilled physicians.

### Application

Another aspect of the present invention provides the use of the above bispecific antibody capable of specifically binding to FGFR2 and PD-1, or the above pharmaceutical composition, in the preparation of a medicament for treating cancer or tumor.

The drug for cancer or tumor according to the present invention refers to a medicament that inhibits and/or treats tumors, which may include delaying the development of tumor-related symptoms and/or reducing the severity of these symptoms, further including alleviating existing tumor-related symptoms and preventing the occurrence of other symptoms, and also includes reducing or preventing tumor metastasis.

The tumors targeted by the medicament of the present invention preferably include but are not limited to: lung cancer, bone cancer, gastric cancer, pancreatic cancer, prostate cancer, skin cancer, head and neck cancer, uterine cancer, ovarian cancer, testicular cancer, fallopian tube cancer, endometrial cancer, cervical cancer, vaginal cancer, vulvar cancer, rectal cancer, colon cancer, anal region cancer, breast cancer, esophageal cancer, small intestine cancer, endocrine system cancer, thyroid cancer, parathyroid cancer, adrenal cancer, urethral cancer, penile cancer, prostate cancer, pancreatic cancer, brain cancer, testicular cancer, lymphoma, transitional cell carcinoma, bladder cancer, kidney cancer, ureteral cancer, renal cell carcinoma, renal pelvis cancer, Hodgkin's lymphoma, non-Hodgkin's lymphoma, soft tissue sarcoma, pediatric solid tumor, lymphocytic lymphoma, central nervous system tumor, primary central nervous system lymphoma, spinal tumor, brainstem glioma, pituitary adenoma, melanoma, Kaposi's sarcoma, epidermoid carcinoma, squamous cell carcinoma, T-cell lymphoma, chronic or acute leukemia, or a combination thereof.

When the bispecific antibody and its composition of the present invention are administered to animals including humans, the dosage varies depending on factors such as the patient's age and weight, the nature and severity of the disease, and the route of administration. Guidance can be taken from the results of animal experiments and other relevant considerations, with the total administered dose typically falling within a defined range. Specifically, the intravenous injection dose is 1-1800 mg/day.

The bispecific antibody and its composition of the present invention can also be administered in combination with other antitumor drugs to achieve more effective tumor treatment. These antitumor drugs include but are not limited to: 1. Cytotoxic drugs: 1) Drugs acting on nucleic acid chemical structures: alkylating agents such as nitrogen mustards, nitrosoureas, methyl sulfonates; platinum compounds such as Cisplatin, Carboplatin, and Oxaliplatin; antibiotics such as Adriamycin/Doxorubicin, Dactinomycin D, Daunorubicin, Epirubicin, Mithramycin; 2) Drugs affecting nucleic acid metabolism: dihydrofolate reductase inhibitors such as Methotrexate and Pemetrexed; thymidine synthase inhibitors such as fluorouracil class (5-fluorouracil, capecitabine); purine nucleotide synthase inhibitors such as 6-mercaptopurine; nucleotide reductase inhibitors such as Hydroxycarbamide; DNA polymerase inhibitors such as Cytosine arabinoside and Gemcitabine; 3) Drugs acting on tubulin: Docetaxel, Vincristine, Vinorelbine, podophyllotoxins, homoharringtonine; 2. Hormonal drugs: antiestrogens such as Tamoxifen, Droloxifene, Exemestane; aromatase inhibitors such as Aminoglutethimide, Formestane, Letrozole, Anastrozole; antiandrogens: Flutamide; RH-LH agonists/antagonists: Zoladex, Enanton, etc.; 3. Biological response modifier drugs: These drugs mainly regulate the body's immune function to achieve antitumor effects, such as Interferons, Interleukin-2, Thymosins; 4. Monoclonal antibody drugs: Trastuzumab, Rituximab, Cetuximab, Bevacizumab. The bispecific antibody and its composition disclosed in the present invention can be used in combination with one or a combination of the above antitumor drugs.

### The main advantages of the present invention include

The beneficial effects of the anti-FGFR2/PD-1 bispecific antibody of the present invention are:
(1) It can regulate T-cell immune activity by binding to the T-cell surface molecule PD-1, and specifically bind to tumor cells expressing FGFR2, inducing immune cells to target and kill FGFR2-positive tumor cells. In particular, it has a significant inhibitory effect on the proliferation of FGFR2-positive tumor cells, and the ADCC effect is also relatively significant;
(2) High specificity and good safety;
(3) High expression level;
(4) Stable structure.

The present invention is further described below in conjunction with specific examples. It should be understood that these examples are intended only to illustrate the present invention, rather than limiting the scope of the present invention. In the following examples, experimental methods without specified detailed conditions are generally carried out under conventional conditions as described in Sambrook et al., Molecular Cloning: A Laboratory Manual (New York: Cold Spring Harbor Laboratory Press, 1989), or as recommended by the manufacturers. Unless otherwise stated, percentages and parts are calculated by weight.

The experimental materials and sources used in the following examples, as well as the preparation methods of experimental reagents, are specifically described as follows.

### Experimental materials:

Competent cells: Sangon Biotech, catalog number B528412.
293F cells: GIBCO, catalog number R79007.
hPBMC: AusaCell, catalog number FPB004F-C-MLR.
0.45 µm filter: Millipore, catalog number SLHV013SL.
SNU-16 cells: Nanjing Kebai, catalog number CBP60502.
Jurkat-PD1-NFAT-luc cells: Promega, catalog number J1252.
CHO-K1-PD-L1 cells: Promega, catalog number J1252.
Ba/F3 cells: Saili, catalog number SLB-CL0019A.
293FT cells: GIBCO, catalog number R70007.
Hitrap Mabselect Sure affinity chromatography column: Cytiva, catalog number 11003493. Waters MassPREP^{™} Micro Desalting Column: Waters, model 186004032.

### Experimental reagents:

Endo-free Plasmid Maxi Kit: Tiangen, catalog number DP117.
DNA Purification and Recovery Kit: Tiangen, catalog number DP214.
ClonExpressTM II One Step Cloning Kit: Vazyme, catalog number C112.
PrimeSTAR^{®} HS DNA Polymerase: Takara, catalog number R010B.
Coating solution: 1.59 g sodium carbonate, 2.93 g sodium bicarbonate, add double-distilled water to a final volume of 1 L.
PBST: PBS+0.05%Tween 20_{∘}
Tween 20: Aladdin, catalog number T104863.
ELISA blocking solution: PBST + 1% BSA.
BSA: Sangon Biotech, catalog number A60332.
Human-FGFR2-his protein: Kactus, catalog number FGR-HM1BD.
HRP-labeled goat anti-human Fc antibody: Biodragon, catalog number BF03031.
TMB: BD Biosciences, catalog number 555214.
Stop solution: 2M sulfuric acid solution.
0.25% Trypsin-EDTA: GIBCO, catalog number 25200-072.
1640 complete medium: RPMI1640 medium + 10% FBS + 1% Pen Strep + 1% sodium pyruvate.
RPMI1640 medium: GIBCO, catalog number 22400089.
FBS: GIBCO, catalog number 10099-141.
Pen Strep: GIBCO, catalog number 1514022.
Sodium pyruvate: GIBCO, catalog number 11360-070.
DMEM complete medium: DMEM high glucose medium + 10% FBS + 1% Pen Strep + 1% GLUMAX.
DMEM high glucose medium: GIBCO, catalog number 11965-092.
GlutaMAX: GIBCO, catalog number 35050-061.
CellTiter-Glo^{®} Luminescent Cell Viability Assay: Promega, catalog number G7572. Goat anti-human Fab-FITC: Thermo Fisher, catalog number MA1-10379.
Glycosidase F: NEB, catalog number P0704L.

### Experimental instruments:

Mastercycler nexus PCR instrument: purchased from Eppendorf.
Hitrap Mabselect Sure column: purchased from Cytiva.
HiLoad 26/600 Superdex 200 pg column: purchased from Cytiva.
SpectraMax i3x microplate reader: purchased from Molecular Devices.
SpectraMax M5 microplate reader: purchased from Molecular Devices.
Coulter CytoFLEX flow cytometer: purchased from Beckman.
FD-0007 automated capillary differential scanning calorimeter: purchased from Malvern. LC/MS liquid chromatography-mass spectrometer: purchased from Waters.

The sequences of the present invention are shown in Table B below:

| SEQ ID NO. | Name | Sequence |
|---|---|---|
| 1 | Amino acid sequence of complementarity determining region HCDR1 in the heavy chain variable region VH of anti-FGFR2 mAb | SYWMH |
| 2 | Amino acid sequence of complementarity determining region HCDR2 in the heavy chain variable region VH of anti-FGFR2 mAb | AIYPGNSDTDYSGKFKG |
| 3 | Amino acid sequence of complementarity determining region HCDR3 in the heavy chain variable region VH of anti-FGFR2 mAb | FDY |
| 4 | Amino acid sequence of complementarity determining region LCDR1 in the light chain variable region VL of anti-FGFR2 mAb | RASSSVNYMYWY |
| 5 | Amino acid sequence of complementarity determining region LCDR2 in the light chain variable region VL of anti-FGFR2 mAb | LTSNRAT |
| 6 | Amino acid sequence of complementarity determining region LCDR3 in the light chain variable region VL of anti-FGFR2 mAb | QQWSSNPYT |
| 7 | Amino acid sequence of complementarity determining region HCDR1 in the heavy chain variable region VH of anti-PD-1 mAb | SYDMS |
| 8 | Amino acid sequence of complementarity determining region HCDR2 in the heavy chain variable region VH of anti-PD-1 mAb | TISGGGRYTYYPDTVKG |
| 9 | Amino acid sequence of complementarity determining region HCDR3 in the heavy chain variable region VH of anti-PD-1 mAb | PYGGYFDV |
| 10 | Amino acid sequence of complementarity determining region LCDR1 in the light chain variable region VL of anti-PD-1 mAb | RASQSISNFLH |
| 11 | Amino acid sequence of complementarity determining region LCDR2 in the light chain variable region VL of anti-PD-1 mAb | YASQSIS |
| 12 | Amino acid sequence of complementarity determining region LCDR3 in the light chain variable region VL of anti-PD-1 mAb | QQSNSWPHT |
| 13 | Amino acid sequence of the heavy chain variable region VH of anti-FGFR2 mAb | QVQLVQSGAEVKKPGSSVKVSCKASGYTFTSYWMHWVRQAPGQGLEWMGAIY PGNSDTDYSGKFKGRVTITADESTSTAYMELSSLRSEDTAVYYCSKFDYWGQGT LVTVSS |
| 14 | Amino acid sequence of the light chain variable region VL of anti-FGFR2 mAb | EIVLTQSPATLSLSPGERATLSCRASSSVNYMYWYQQKPGQAPRPWIYLTSNRAT GVPARFSGSGSGTDYTLTISSLEPEDFAVYYCQQWSSNPYTFGQGTKLEIKRTV |
| 15 | Amino acid sequence of the heavy chain variable region VH of anti-PD-1 mAb | EVKLVESGGGLVQPGGSLRLSCAASGFAFSSYDMSWVRQAPGKRLEWVATISGG GRYTYYPDTVKGRFTISRDNAKNSHYLQMNSLRAEDTAVYFCASPYGGYFDVW GQGTLVTVSS |
| 16 | Amino acid sequence of the light chain variable region VL of anti-PD-1 mAb | EIVLTQSPATLSLSPGERATLSCRASQSISNFLHWYQQKPGQAPRLLIKYASQSISG IPARFSGSGSGTDFTLTISSLEPEDFAVYFCQQSNSWPHTFGQGTKVEIK |
| 17 | Amino acid sequence of peptide linker L1 | GGGGSGGGGSGGGGSGGGGS |
| 18 | Amino acid sequence of peptide linker L2 | GGGGSGGGGSGGGGS |
| 19 | Amino acid sequence of heavy chain constant region CH1 | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKV |
| 20 | Amino acid sequence of heavy chain constant region CH2 | PAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISK |
| 21 | Amino acid sequence of heavy chain constant region CH3 | AKGQPREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSLSLSPG K |
| 22 | Amino acid sequence of heavy chain constant region CH2-S239D-I332E | PAPELLGGPDVFLFPPKPKDTLMISRTPEVTCVVVDVSHEDPEVKFNWYVDGVEV HNAKTKPREEQYNSTYRVVSVLTVLHQDWLNGKEYKCKVSNKALPAPEEKTISK |
| 23 | Amino acid sequence of heavy chain constant region CH | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTI SKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLS PGK |
| 24 | Amino acid sequence of heavy chain constant region CH-S239D-I332E | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCP PCPAPELLGGPDVFLFPPKPKDTLMISRTPEVTCVVVDVSHEDPEVKFNWYVDGV EVHNAKTKPREEQYNSTYRVVSVLTVLHQDWLNGKEYKCKVSNKALPAPEEKTI SKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLS PGK |
| 25 | Amino acid sequence of anti-FGFR2 mAb heavy chain | QVQLVQSGAEVKKPGSSVKVSCKASGYTFTSYWMHWVRQAPGQGLEWMGAIY PGNSDTDYSGKFKGRVTITADESTSTAYMELSSLRSEDTAVYYCSKFDYWGQGT LVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPA PIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKS LSLSPGK |
| 26 | Amino acid sequence of anti-FGFR2 mAb heavy chain HC-S239D-I332E | QVQLVQSGAEVKKPGSSVKVSCKASGYTFTSYWMHWVRQAPGQGLEWMGAIY PGNSDTDYSGKFKGRVTITADESTSTAYMELSSLRSEDTAVYYCSKFDYWGQGT LVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCD KTHTCPPCPAPELLGGPDVFLFPPKPKDTLMISRTPEVTCVVVDVSHEDPEVKFN WYVDGVEVHNAKTKPREEQYNSTYRVVSVLTVLHQDWLNGKEYKCKVSNKAL PAPEEKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALHNHYT QKSLSLSPGK |
| 27 | Amino acid sequence of anti-PD-1 mAb single-chain variable fragment scFv-VLVH | EIVLTQSPATLSLSPGERATLSCRASQSISNFLHWYQQKPGQAPRLLIKYASQSISG IPARFSGSGSGTDFTLTISSLEPEDFAVYFCQQSNSWPHTFGQGTKVEIKGGGGSG GGGSGGGGSGGGGSEVKLVESGGGLVQPGGSLRLSCAASGFAFSSYDMSWVRQ APGKRLEWVATISGGGRYTYYPDTVKGRFTISRDNAKNSHYLQMNSLRAEDTAV YFCASPYGGYFDVWGQGTLVTVSS |
| 28 | Amino acid sequence of the light chain of anti-FGFR2/PD-1 bispecific antibody a/b | EIVLTQSPATLSLSPGERATLSCRASSSVNYMYWYQQKPGQAPRPWIYLTSNRAT GVPARFSGSGSGTDYTLTISSLEPEDFAVYYCQQWSSNPYTFGQGTKLEIKRTVA APSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC |
| 29 | Amino acid sequence of the heavy chain of anti-FGFR2/PD-1 bispecific antibody a | QVQLVQSGAEVKKPGSSVKVSCKASGYTFTSYWMHWVRQAPGQGLEWMGAIY PGNSDTDYSGKFKGRVTITADESTSTAYMELSSLRSEDTAVYYCSKFDYWGQGT LVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCD KTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPA PIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKS LSLSPGKGGGGSGGGGSGGGGSEIVLTQSPATLSLSPGERATLSCRASQSISNFLH WYQQKPGQAPRLLIKYASQSISGIPARFSGSGSGTDFTLTISSLEPEDFAVYFCQQS NSWPHTFGQGTKVEIKGGGGSGGGGSGGGGSGGGGSEVKLVESGGGLVQPGGS LRLSCAASGFAFSSYDMSWVRQAPGKRLEWVATISGGGRYTYYPDTVKGRFTIS RDNAKNSHYLQMNSLRAEDTAVYFCASPYGGYFDVWGQGTLVTVSS |
| 30 | Amino acid sequence of the heavy chain of anti-FGFR2/PD-1 bispecific antibody b | QVQLVQSGAEVKKPGSSVKVSCKASGYTFTSYWMHWVRQAPGQGLEWMGAIY PGNSDTDYSGKFKGRVTITADESTSTAYMELSSLRSEDTAVYYCSKFDYWGQGT LVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCD KTHTCPPCPAPELLGGPDVFLFPPKPKDTLMISRTPEVTCVVVDVSHEDPEVKFN WYVDGVEVHNAKTKPREEQYNSTYRVVSVLTVLHQDWLNGKEYKCKVSNKAL PAPEEKTISKAKGQPREPQVYTLPPSREEMTKNQVSLTCLVKGFYPSDIAVEWES NGQPENNYKTTPPVLDSDGSFFLYSKLTVDKSRWQQGNVFSCSVMHEALHNHYT QKSLSLSPGKGGGGSGGGGSGGGGSEIVLTQSPATLSLSPGERATLSCRASQSISN FLHWYQQKPGQAPRLLIKYASQSISGIPARFSGSGSGTDFTLTISSLEPEDFAVYFC QQSNSWPHTFGQGTKVEIKGGGGSGGGGSGGGGSGGGGSEVKLVESGGGLVQP GGSLRLSCAASGFAFSSYDMSWVRQAPGKRLEWVATISGGGRYTYYPDTVKGRF TISRDNAKNSHYLQMNSLRAEDTAVYFCASPYGGYFDVWGQGTLVTVSS |
| 31 | Nucleic acid sequence of the heavy chain of anti-FGFR2/PD-1 bispecific antibody a | CAGGTGCAGCTGGTGCAGTCCGGCGCCGAGGTGAAGAAGCCTGGCTCCAGCG TGAAGGTGTCTTGCAAGGCCAGCGGCTACACCTTCACCTCCTACTGGATGCAC TGGGTGAGGCAGGCCCCCGGCCAGGGCCTGGAGTGGATGGGCGCCATCTACC CTGGCAACAGCGACACCGACTATTCCGGCAAGTTCAAGGGCCGGGTGACCAT CACCGCCGACGAGTCCACCTCCACCGCCTACATGGAGCTGAGCAGCCTGAGG TCCGAGGACACCGCCGTGTACTACTGCAGCAAGTTCGACTACTGGGGCCAGG GCACCCTGGTGACCGTGTCCTCCGCGAGCACCAAGGGACCTTCCGTGTTTCCC CTCGCCCCCAGCTCCAAAAGCACCAGCGGCGGAACAGCTGCTCTCGGCTGTCT CGTCAAGGATTACTTCCCCGAGCCCGTGACCGTGAGCTGGAACAGCGGAGCC CTGACAAGCGGCGTCCACACCTTCCCTGCTGTCCTACAGTCCTCCGGACTGTA CAGCCTGAGCAGCGTGGTGACAGTCCCTAGCAGCTCCCTGGGCACCCAGACA TATATTTGCAACGTGAATCACAAGCCCAGCAACACCAAGGTCGATAAGAAGG TGGAGCCTAAGTCCTGCGACAAGACCCACACATGTCCCCCCTGTCCCGCTCCT GAACTGCTGGGAGGCCCTTCCGTGTTCCTGTTCCCCCCTAAGCCCAAGGACAC CCTGATGATTTCCAGGACACCCGAGGTGACCTGTGTGGTGGTGGACGTCAGCC ACGAGGACCCCGAGGTGAAATTCAACTGGTACGTCGATGGCGTGGAGGTGCA CAACGCTAAGACCAAGCCCAGGGAGGAGCAGTACAATTCCACCTACAGGGTG GTGTCCGTGCTGACCGTCCTCCATCAGGACTGGCTGAACGGCAAAGAGTATAA GTGCAAGGTGAGCAACAAGGCCCTCCCTGCTCCCATCGAGAAGACCATCAGC AAAGCCAAGGGCCAGCCCAGGGAACCTCAAGTCTATACCCTGCCTCCCAGCA GGGAGGAGATGACCAAGAACCAAGTGAGCCTCACATGCCTCGTCAAGGGCTT CTATCCTTCCGATATTGCCGTCGAGTGGGAGTCCAACGGACAGCCCGAGAACA ACTACAAGACAACACCCCCCGTGCTCGATTCCGATGGCAGCTTCTTCCTGTAC TCCAAGCTGACCGTGGACAAGTCCAGATGGCAACAAGGCAACGTCTTCAGTT GCAGCGTCATGCATGAGGCCCTCCACAACCACTACACCCAGAAGAGCCTCTC CCTGAGCCCTGGAAAGGGCGGTGGGGGTAGTGGAGGCGGAGGTTCGGGAGG GGGCGGTAGCGAGATCGTCCTTACCCAATCACCAGCAACCTTGTCACTGTCAC CAGGTGAAAGAGCAACCCTCAGTTGTAGGGCTAGTCAGAGTATCTCCAACTTC CTGCACTGGTACCAGCAGAAGCCTGGACAGGCCCCTCGGTTGCTCATTAAGTA CGCCTCTCAATCTATCAGCGGAATCCCCGCTCGCTTTTCTGGCTCTGGCTCCGG GACTGATTTCACTCTGACAATTTCCAGCCTGGAACCCGAGGACTTTGCCGTTT ATTTTTGCCAGCAGAGCAATAGCTGGCCCCATACATTCGGGCAGGGCACAAA AGTGGAGATAAAAGGTGGAGGCGGTTCAGGCGGAGGTGGCAGCGGCGGTGG CGGGTCGGGAGGGGGTGGCTCTGAAGTCAAACTCGTGGAGTCCGGCGGAGGC CTCGTTCAACCAGGTGGATCTCTTCGTTTGTCCTGCGCAGCATCAGGATTCGCT TTCTCCAGCTACGACATGAGCTGGGTCCGACAGGCTCCTGGAAAGAGGCTGG AATGGGTTGCTACTATCAGCGGCGGTGGTAGATATACTTATTACCCCGATACC GTAAAGGGGAGGTTCACCATTAGTCGCGATAACGCCAAAAATTCACACTACC TGCAGATGAACTCTCTGCGGGCCGAGGACACCGCCGTGTACTTTTGTGCCAGT CCCTATGGCGGGTATTTTGACGTGTGGGGCCAGGGGACACTGGTGACTGTGAG TTCT |
| 32 | Nucleic acid sequence of the heavy chain of anti-FGFR2/PD-1 bispecific antibody b | CAGGTGCAGCTGGTGCAGTCCGGCGCCGAGGTGAAGAAGCCTGGCTCCAGCG TGAAGGTGTCTTGCAAGGCCAGCGGCTACACCTTCACCTCCTACTGGATGCAC TGGGTGAGGCAGGCCCCCGGCCAGGGCCTGGAGTGGATGGGCGCCATCTACC CTGGCAACAGCGACACCGACTATTCCGGCAAGTTCAAGGGCCGGGTGACCAT CACCGCCGACGAGTCCACCTCCACCGCCTACATGGAGCTGAGCAGCCTGAGG TCCGAGGACACCGCCGTGTACTACTGCAGCAAGTTCGACTACTGGGGCCAGG GCACCCTGGTGACCGTGTCCTCCGCGAGCACCAAGGGACCTTCCGTGTTTCCC CTCGCCCCCAGCTCCAAAAGCACCAGCGGCGGAACAGCTGCTCTCGGCTGTCT CGTCAAGGATTACTTCCCCGAGCCCGTGACCGTGAGCTGGAACAGCGGAGCC CTGACAAGCGGCGTCCACACCTTCCCTGCTGTCCTACAGTCCTCCGGACTGTA CAGCCTGAGCAGCGTGGTGACAGTCCCTAGCAGCTCCCTGGGCACCCAGACA TATATTTGCAACGTGAATCACAAGCCCAGCAACACCAAGGTCGATAAGAAGG TGGAGCCTAAGTCCTGCGACAAGACCCACACATGTCCCCCCTGTCCCGCTCCT GAACTGCTGGGAGGCCCTgaCGTGTTCCTGTTCCCCCCTAAGCCCAAGGACAC CCTGATGATTTCCAGGACACCCGAGGTGACCTGTGTGGTGGTGGACGTCAGCC ACGAGGACCCCGAGGTGAAATTCAACTGGTACGTCGATGGCGTGGAGGTGCA CAACGCTAAGACCAAGCCCAGGGAGGAGCAGTACAATTCCACCTACAGGGTG GTGTCCGTGCTGACCGTCCTCCATCAGGACTGGCTGAACGGCAAAGAGTATAA GTGCAAGGTGAGCAACAAGGCCCTCCCTGCTCCCgagGAGAAGACCATCAGCA AAGCCAAGGGCCAGCCCAGGGAACCTCAAGTCTATACCCTGCCTCCCAGCAG GGAGGAGATGACCAAGAACCAAGTGAGCCTCACATGCCTCGTCAAGGGCTTC TATCCTTCCGATATTGCCGTCGAGTGGGAGTCCAACGGACAGCCCGAGAACA ACTACAAGACAACACCCCCCGTGCTCGATTCCGATGGCAGCTTCTTCCTGTAC TCCAAGCTGACCGTGGACAAGTCCAGATGGCAACAAGGCAACGTCTTCAGTT GCAGCGTCATGCATGAGGCCCTCCACAACCACTACACCCAGAAGAGCCTCTC CCTGAGCCCTGGAAAGGGCGGTGGGGGTAGTGGAGGCGGAGGTTCGGGAGG GGGCGGTAGCGAGATCGTCCTTACCCAATCACCAGCAACCTTGTCACTGTCAC CAGGTGAAAGAGCAACCCTCAGTTGTAGGGCTAGTCAGAGTATCTCCAACTTC CTGCACTGGTACCAGCAGAAGCCTGGACAGGCCCCTCGGTTGCTCATTAAGTA CGCCTCTCAATCTATCAGCGGAATCCCCGCTCGCTTTTCTGGCTCTGGCTCCGG GACTGATTTCACTCTGACAATTTCCAGCCTGGAACCCGAGGACTTTGCCGTTT ATTTTTGCCAGCAGAGCAATAGCTGGCCCCATACATTCGGGCAGGGCACAAA AGTGGAGATAAAAGGTGGAGGCGGTTCAGGCGGAGGTGGCAGCGGCGGTGG CGGGTCGGGAGGGGGTGGCTCTGAAGTCAAACTCGTGGAGTCCGGCGGAGGC CTCGTTCAACCAGGTGGATCTCTTCGTTTGTCCTGCGCAGCATCAGGATTCGCT TTCTCCAGCTACGACATGAGCTGGGTCCGACAGGCTCCTGGAAAGAGGCTGG AATGGGTTGCTACTATCAGCGGCGGTGGTAGATATACTTATTACCCCGATACC GTAAAGGGGAGGTTCACCATTAGTCGCGATAACGCCAAAAATTCACACTACC TGCAGATGAACTCTCTGCGGGCCGAGGACACCGCCGTGTACTTTTGTGCCAGT CCCTATGGCGGGTATTTTGACGTGTGGGGCCAGGGGACACTGGTGACTGTGAG TTCT |
| 33 | Nucleic acid sequence of the light chain of anti-FGFR2/PD-1 bispecific antibody a/b | GAGATCGTGCTGACCCAGTCCCCTGCCACCCTGTCCCTGTCCCCCGGCGAGCG GGCCACCCTGTCTTGCCGGGCTAGCTCCTCCGTGAACTACATGTACTGGTACC AGCAGAAGCCTGGCCAGGCCCCTAGGCCCTGGATCTACCTGACCAGCAACAG GGCCACCGGCGTGCCCGCCAGGTTCTCCGGCAGCGGCTCCGGCACCGACTAC ACCCTGACCATCTCCTCCCTGGAGCCCGAGGACTTCGCCGTGTACTACTGCCA GCAGTGGTCTAGCAATCCCTACACTTTCGGCCAGGGCACCAAGCTGGAGATCA AGAGGACCGTGGCCGCCCCCTCCGTGTTCATTTTTCCCCCTAGCGACGAGCAG CTGAAGAGCGGCACCGCTAGCGTGGTGTGCCTGCTGAACAACTTCTACCCCAG GGAGGCCAAGGTGCAGTGGAAAGTGGACAACGCCCTGCAGAGCGGCAACTCC CAGGAGTCCGTGACCGAGCAGGACAGCAAGGACAGCACCTACTCCCTGTCCT CCACCCTGACCCTGTCCAAGGCCGACTACGAGAAGCACAAGGTGTACGCCTG TGAGGTGACACACCAGGGCCTGTCCTCCCCCGTGACAAAGTCCTTCAACAGGG GCGAGTGC |

### Example 1. Construction of FGFR2/PD-1 bispecific antibody molecules

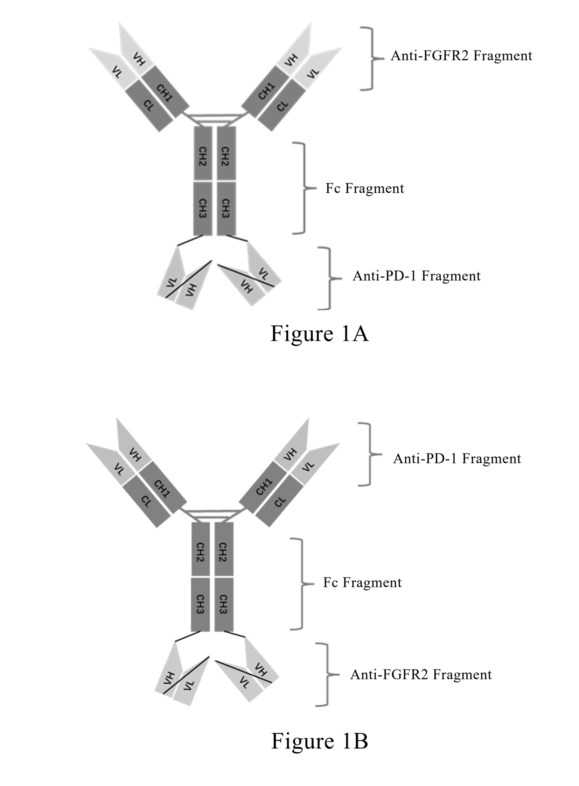

The present invention adopts the method of tandemly linking an anti-FGFR2 monoclonal antibody IgG and an anti-PD-1 monoclonal antibody scFv to construct anti-FGFR2/PD-1 bispecific antibodies, the structure of which is shown in Figure 1A.

Among them, the anti-FGFR2 monoclonal antibody sequence in the anti-FGFR2/PD-1 bispecific antibody is derived from the humanized monoclonal antibody disclosed in patent CN102905723A, serving as the positive control for the anti-FGFR2 monoclonal antibody. The anti-PD-1 monoclonal antibody sequence in the anti-FGFR2/PD-1 bispecific antibody is derived from the humanized monoclonal antibody disclosed in patent CN201710054783.5, serving as the positive control for the anti-PD-1 monoclonal antibody.

The scFv-VLVH of the anti-FGFR2/PD-1 bispecific antibody is obtained by connecting the anti-PD-1 light chain variable region VL (SEQ ID NO. 16) and the anti-PD-1 heavy chain variable region VH (SEQ ID NO. 15) via the peptide linker L1 (SEQ ID NO. 17), resulting in VL-L1-VH, i.e., PD-1-scFv-VLVH (SEQ ID NO. 27).

The heavy chain of anti-FGFR2/PD-1 bispecific antibody a (SEQ ID NO. 29) is obtained by connecting the C-terminus of the anti-FGFR2 monoclonal antibody heavy chain (SEQ ID NO. 25) and the N-terminus of the anti-PD-1 monoclonal antibody variable fragment PD-1-scFv-VLVH (SEQ ID NO. 27) via the peptide linker L2 (SEQ ID NO. 18), while the light chain of the anti-FGFR2 monoclonal antibody (SEQ ID NO. 28) remains unchanged. Corresponding sites on human IgG1 were selected to mutate the heavy chain constant region of the anti-FGFR2 monoclonal antibody. After screening, S239D and I332E were selected to obtain the anti-FGFR2 monoclonal antibody heavy chain HC-S239D-I332E.

The heavy chain of anti-FGFR2/PD-1 bispecific antibody b (SEQ ID NO. 30) is obtained by connecting the N-terminus of the anti-FGFR2 monoclonal antibody heavy chain HC-S239D-I332E (SEQ ID NO. 26) and the anti-PD-1 monoclonal antibody variable fragment PD-1-scFv-VLVH (SEQ ID NO. 27) via the peptide linker L2 (SEQ ID NO. 18), while the light chain of the anti-FGFR2 monoclonal antibody (SEQ ID NO. 28) remains unchanged. Meanwhile, during the construction of the anti-FGFR2/PD-1 bispecific antibody molecules, different linkage methods and configurations were designed to connect the anti-FGFR2 monoclonal antibody IgG or its scFv and the anti-PD-1 monoclonal antibody IgG or its scFv, forming different forms of anti-FGFR2/PD-1 bispecific antibody molecules.

The anti-FGFR2 heavy chain variable region VH (SEQ ID NO. 13) and the anti-FGFR2 light chain variable region VH (SEQ ID NO. 14) are connected via the peptide linker L1 (SEQ ID NO. 17) to obtain VH-L1-VL, i.e., FGFR2-scFv-VHVL. The heavy chain of anti-FGFR2/PD-1 bispecific antibody c is obtained by connecting the C-terminus of the anti-PD-1 monoclonal antibody heavy chain and the N-terminus of the anti-FGFR2 monoclonal antibody variable fragment FGFR2-scFv-VHVL via the peptide linker L2 (SEQ ID NO. 18), while the light chain of the anti-PD-1 monoclonal antibody remains unchanged. The structure is shown in Figure 1B.

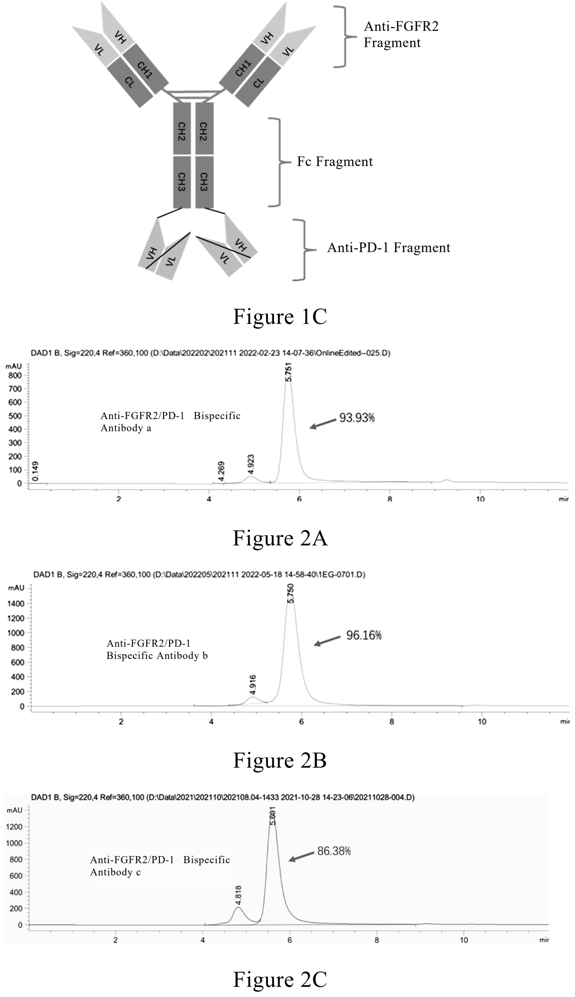

The anti-PD-1 heavy chain variable region VH (SEQ ID NO. 15) and the anti-PD-1 light chain variable region VL (SEQ ID NO. 16) are connected via the peptide linker L1 (SEQ ID NO. 17) to obtain VH-L1-VL, i.e., PD-1-scFv-VHVL. The heavy chain of anti-FGFR2/PD-1 bispecific antibody d is obtained by connecting the C-terminus of the anti-FGFR2 monoclonal antibody heavy chain (SEQ ID NO. 25) and the N-terminus of the anti-PD-1 monoclonal antibody variable fragment PD-1-scFv-VHVL via the peptide linker L2 (SEQ ID NO. 18), while the light chain of the anti-FGFR2 monoclonal antibody (SEQ ID NO. 28) remains unchanged. The structure is shown in Figure 1C.

To improve the expression efficiency of the antibody molecules in CHO cells, the nucleic acid sequences of the anti-FGFR2-PD1 bispecific antibody molecules were codonoptimized, primarily considering factors such as codon preference, GC content, mRNA secondary structure, and repetitive sequences. The nucleic acid sequences of the antibodies were synthesized by Sangon Biotech Co., Ltd. Amino acid and nucleic acid sequences are shown in the Sequence Listing.

### Example 2. Expression and purification of anti-FGFR2/PD-1 bispecific antibodies

The DNA fragments encoding the heavy and light chains of anti-FGFR2/PD-1 bispecific antibodies a and b were subcloned into the pcDNA3.4 vector. Recombinant plasmids were extracted and co-transfected into 293F cells and/or CHO cells. After culturing the cells for 5-7 days, the culture medium was subjected to high-speed centrifugation and filtered through a 0.22 µm filter before loading onto a Hitrap Mabselect Sure affinity chromatography column. The protein was eluted in one step using 100 mM citrate elution buffer, pH 3.5. The target samples were collected and dialyzed into PBS, pH 7.4. The purified protein was analyzed by UPLC-SEC.

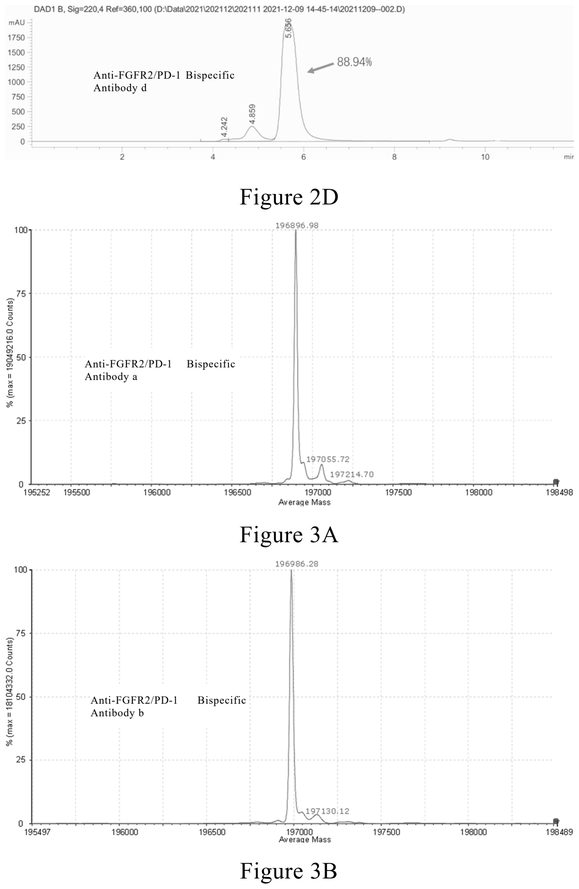

The detection results of anti-FGFR2/PD-1 bispecific antibodies a, b, c, and d are shown in Figures 2A, 2B, 2C, and 2D, respectively. The antibody proteins were homogeneous. The monomer purity of anti-FGFR2/PD-1 bispecific antibody a reached over 93%, while the monomer purity of anti-FGFR2/PD-1 bispecific antibody b was slightly higher than that of bispecific antibody a, reaching over 96%. The purity of anti-FGFR2/PD-1 bispecific antibodies c and d was slightly lower than that of bispecific antibody a, with monomer purities above 86% and 88%, respectively.

### Example 3. Detection of de-N-glycosylated intact molecular weight of anti-FGFR2/PD-1 bispecific antibodies

1 mg each of anti-FGFR2/PD-1 bispecific antibodies a and b were desalted into ultrapure water using ultrafiltration and concentrated to above 20 mg/mL. 60 µL of sample was taken, supplemented with 59 µL of ultrapure water, and then 1 µL of Glycosidase F was added. After incubation in a 37°C water bath for 2 hours, the de-glycosylated intact molecular weights of the anti-FGFR2/PD-1 bispecific antibody a and b samples were detected using an LC/MS system equipped with a Waters MassPREP^{™} Micro Desalting Column.

The experimental results are shown in Figures 3A and 3B. The theoretical de-glycosylated molecular weight of anti-FGFR2/PD-1 bispecific antibody a is 196892.05 Da, and the measured molecular weight after mass spectrometry analysis is 196896.98 Da. The theoretical de-glycosylated molecular weight of anti-FGFR2/PD-1 bispecific antibody b is 196979.98 Da, and the measured molecular weight after mass spectrometry analysis is 196986.28 Da. The theoretical molecular weights of anti-FGFR2/PD-1 bispecific antibodies a and b closely match the measured molecular weights.

### Example 4. Determination of thermal stability of Anti-FGFR2/PD-1 bispecific antibodies

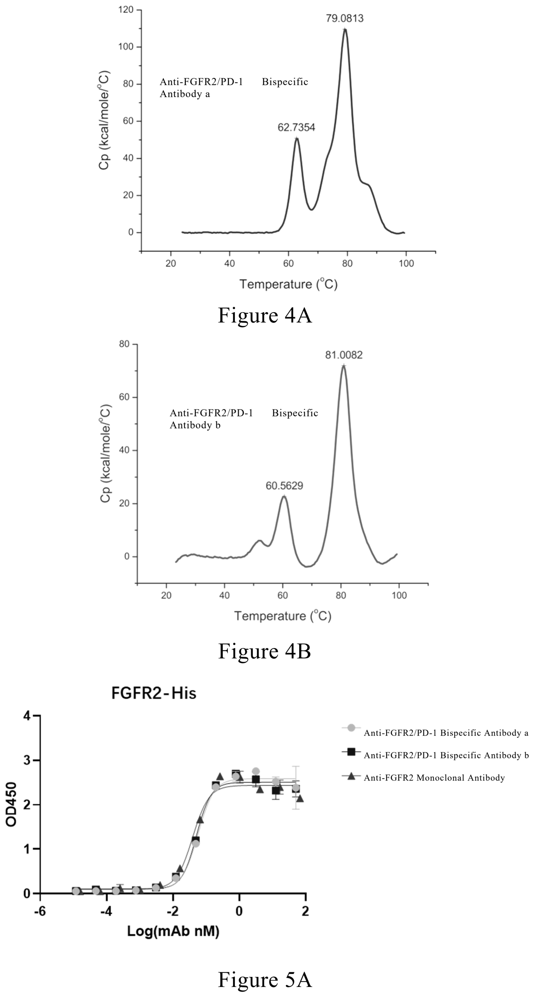

Differential Scanning Calorimetry (DSC) was used to identify the changes in molar heat capacity of the anti-FGFR2/PD-1 bispecific antibodies with temperature to evaluate their thermal stability. The protein solutions of anti-FGFR2/PD-1 bispecific antibodies a and b were diluted to 0.5 mg/mL with 1×PBS (pH 7.4) buffer. 500 µL of each sample was taken and analyzed using an automated capillary differential scanning calorimeter. The obtained DSC thermograms are shown in Figures 4A and 4B.

The experimental results show that as the temperature increases, the Tm₁ and Tm₂ of FGFR2/PD-1 bispecific antibody a are at 62.74°C and 79.08°C, respectively, while the Tm₁ and Tm₂ of FGFR2/PD-1 bispecific antibody b are at 60.56°C and 81.01°C, respectively. Both anti-FGFR2-PD1 bispecific antibodies a and b showed the first endothermic peak at approximately 60°C and the second endothermic peak at approximately 80°C, indicating that the thermal stability of the anti-FGFR2/PD-1 bispecific antibodies a and b proteins is comparable.

### Example 5. Determination of antigen affinity of bispecific antibodies by enzyme-linked immunosorbent assay (ELISA)

### 5.1 Detection of affinity of anti-FGFR2/PD-1 bispecific antibodies for FGFR2

Recombinant Human-FGFR2-His protein was diluted to 200 ng/mL with coating buffer and added to the microplate at 100 µL/well, then left at room temperature for 2 hours or at 4°C overnight. The coating solution was removed (residual droplets were removed with absorbent paper), and blocking buffer was added to the microplate at 200 µL/well, then left at room temperature for 1-2 hours. The blocking solution was removed (residual droplets were removed with absorbent paper). Anti-FGFR2/PD-1 bispecific antibodies a and b, as well as the anti-FGFR2 monoclonal antibody, were diluted with blocking buffer to 10 µg/mL and then subjected to four-fold serial dilution to create 12 concentration gradients(highest concentration 10 µg/mL, lowest concentration 0.002 ng/mL). These were added sequentially to the blocked microplate at 100 µL/well and left at room temperature for 1 hour. The plate was washed 3 times with PBST (residual droplets were removed with absorbent paper). HRP-labeled goat anti-human Fc antibody was diluted 1:3000 with blocking buffer and added to the microplate at 100 µL/well, then left at room temperature for 30 minutes. The plate was washed 3 times with PBST (residual droplets were removed with absorbent paper). TMB substrate solution was added at 100 µL/well and left at room temperature in the dark for 5 minutes. Stop solution was added at 70 µL/well to terminate the substrate color reaction. The OD value at 450 nm was read using a microplate reader. Data were analyzed using GraphPad, plotted, and the EC₅₀ was calculated.

The experimental results are shown in Figure 5A. The EC₅₀ values (unit: nM) for the binding of anti-FGFR2/PD-1 bispecific antibody molecules a and b, and the positive control anti-FGFR2 monoclonal antibody to Human-FGFR2-His were 0.0568, 0.0512, and 0.0392, respectively, indicating comparable affinity among the three.

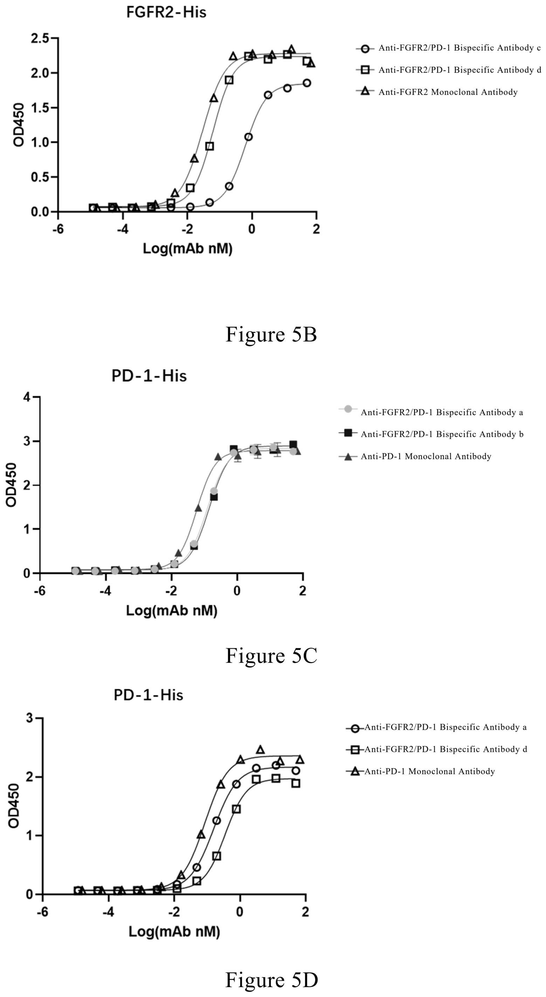

The affinity of anti-FGFR2/PD-1 bispecific antibody molecules c and d, and the positive control anti-FGFR2 monoclonal antibody to Human-FGFR2-His was determined using the same method. The experimental results are shown in Figure 5B. The EC₅₀ values (unit: nM) were 0.6244, 0.0615, and 0.0297, respectively. The affinity of anti-FGFR2/PD-1 bispecific antibody d was comparable to that of the positive control anti-FGFR2 monoclonal antibody, while the affinity of anti-FGFR2/PD-1 bispecific antibody c was significantly reduced compared to the positive control anti-FGFR2 monoclonal antibody.

The structures of anti-FGFR2/PD-1 bispecific antibodies a, b, and d are similar, all being in the form of anti-FGFR2 monoclonal antibody IgG tandemly linked with anti-PD-1 monoclonal antibody scFv. In contrast, anti-FGFR2/PD-1 bispecific antibody c is in the form of anti-PD-1 monoclonal antibody IgG tandemly linked with anti-FGFR2 monoclonal antibody scFv. The affinity of bispecific antibodies a and b was superior to that of bispecific antibody c, indicating that the bispecific antibody molecule formed by tandemly linking anti-FGFR2 monoclonal antibody IgG with anti-PD-1 monoclonal antibody scFv exhibits higher affinity for binding to the FGFR2 antigen.

### 5.2 Detection of affinity of anti-FGFR2/PD-1 bispecific antibodies for PD-1

Recombinant Human-PD-1-His protein was diluted to 200 ng/mL with coating buffer and added to the microplate at 100 µL/well, then left at room temperature for 2 hours or at 4°C overnight. The coating solution was removed (residual droplets were removed with absorbent paper), and blocking buffer was added to the microplate at 200 µL/well, then left at room temperature for 1-2 hours. The blocking solution was removed (residual droplets were removed with absorbent paper). Anti-FGFR2/PD-1 bispecific antibodies a and b, as well as the anti-PD-1 monoclonal antibody, were diluted with blocking buffer to 10 µg/mL and then subjected to four-fold serial dilution to create 12 concentration gradients (highest concentration 10 µg/mL, lowest concentration 0.002 ng/mL). These were added sequentially to the blocked microplate at 100 µL/well and left at room temperature for 1 hour. The plate was washed 3 times with PBST (residual droplets were removed with absorbent paper). HRP-labeled goat anti-human Fc antibody was diluted 1:3000 with blocking buffer and added to the microplate at 100 µL/well, then left at room temperature for 30 minutes. The plate was washed 3 times with PBST (residual droplets were removed with absorbent paper). TMB substrate solution was added at 100 µL/well and left at room temperature in the dark for 5 minutes. Stop solution was added at 70 µL/well to terminate the substrate color reaction. The OD value at 450 nm was read using a microplate reader. Data were analyzed using GraphPad, plotted, and the EC₅₀ was calculated.

The experimental results are shown in Figure 5C. The EC₅₀ values (unit: nM) for the binding of anti-FGFR2/PD-1 bispecific antibody molecules a and b, and the positive control anti-PD-1 monoclonal antibody to Human-PD-1-His were 0.1216, 0.1411, and 0.0586, respectively. The affinity of bispecific antibodies a and b was reduced compared to that of the monoclonal antibody, indicating that the bispecific antibody formed by linking the variable region of the anti-PD-1 monoclonal antibody in a VL-VH configuration and then connecting it to the heavy chain of the anti-FGFR2 monoclonal antibody exhibits a slightly reduced binding capacity to the PD-1 antigen.

The affinity of anti-FGFR2/PD-1 bispecific antibody molecules a and d, and the positive control anti-PD-1 monoclonal antibody to Human-PD-1-His was determined using the same method. The experimental results are shown in Figure 5D. The EC50 values (unit: nM) were 0.1599, 0.3564, and 0.0856, respectively. The affinity of anti-FGFR2/PD-1 bispecific antibody a for Human-PD-1-His was slightly higher than that of anti-FGFR2/PD-1 bispecific antibody d, indicating that the bispecific antibody molecule formed by linking the variable region of the anti-PD-1 monoclonal antibody in a VL-VH configuration and then tandemly linking it with the anti-FGFR2 monoclonal antibody scFv exhibits higher affinity for binding to the PD-1 antigen.

### Example 6. Blocking activity of anti-FGFR2/PD-1 bispecific antibodies on PD-1/PD-L1 binding

Jurkat-PD-1-NFAT-luc cells expressing PD-1 on the cell surface were used as target cells. The binding affinity of anti-FGFR2/PD-1 bispecific antibodies to these cells was determined by flow cytometry. The cell density was adjusted to 1.5×10⁶ cells/mL with DPBS containing 1% BSA, and 100 µL/well was added to a 96-well plate. Anti-FGFR2/PD-1 bispecific antibodies a and b, and the positive control anti-PD-1 monoclonal antibody were diluted to 200 nM with DPBS containing 1% BSA, followed by four-fold serial dilution to create 12 concentration gradients. 100 µL/well was added to the 96-well plate and mixed thoroughly with Jurkat-PD-1-NFAT-luc cells. Incubated at 4°C for 1 h. Centrifuged at 400 g for 5 min, and the supernatant was discarded. Washed twice with pre-cooled DPBS to remove unbound test antibodies. Added 100 µL/well of goat anti-human Fab-FITC (diluted 800:1), and incubated at 4°C for 1 h. Centrifuged at 400 g for 5 min, and the supernatant was discarded. Washed twice with pre-cooled DPBS, finally resuspended in 200 µL of DPBS, and the binding affinity of anti-FGFR2/PD-1 bispecific antibodies to the cells was determined using a Beckman Coulter CytoFLEX flow cytometer. The obtained data were plotted and analyzed using GraphPad Prism6.

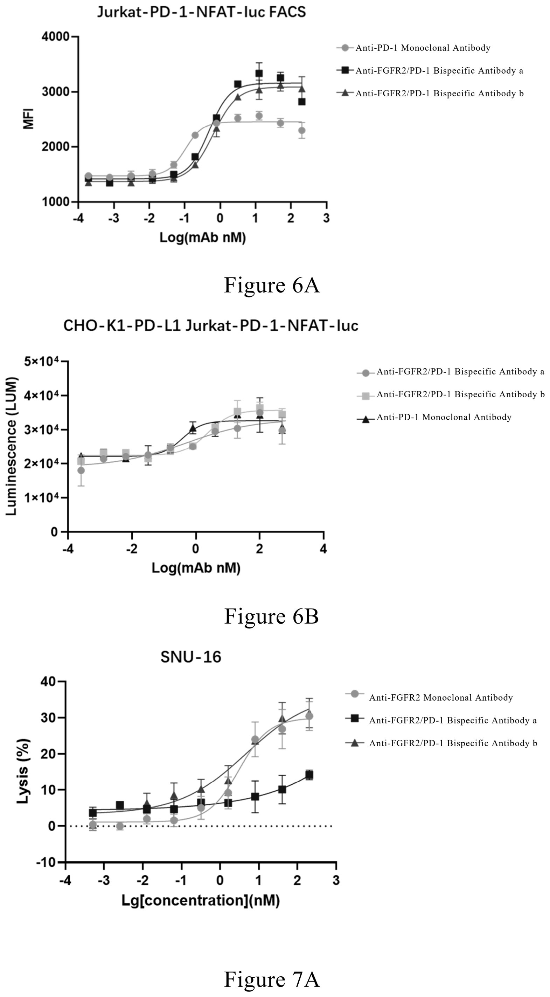

The experimental results are shown in Figure 6A. Anti-FGFR2/PD-1 bispecific antibodies a and b could specifically bind to PD-1 expressed on the cell surface. The EC₅₀ values for the binding of anti-FGFR2/PD-1 bispecific antibodies a and b, and the positive control anti-PD-1 monoclonal antibody to Jurkat-PD1-NFAT-luc cells were 0.4823, 0.6344, and 0.1046 nM, respectively, indicating that the activity of anti-FGFR2/PD-1 bispecific antibodies a and b in binding to cell surface PD-1 protein was relatively weaker compared to the anti-PD-1 monoclonal antibody.

CHO-K1-PD-L1 cells were diluted to 4×10⁵ cells/mL with complete medium, added to a 96-well plate (model 3903) at 100 µL/well, and incubated overnight in an incubator at 37°C with 5% CO₂. The medium was then removed and the plate was set aside for use. Anti-FGFR2/PD-1 bispecific antibody molecules a and b, and the anti-PD-1 monoclonal antibody were diluted to 500 nM with assay medium (RPMI 1640 + 1% FBS), and subjected to fivefold serial dilution to create 10 concentrations (highest concentration 500 nM, lowest concentration 2.56×10⁻⁴ nM). 40 µL/well was added to the 96-well plate containing CHO-K1-PD-L1 cells. Jurkat-PD-1-NFAT-luc cells were diluted to 4×10⁵ cells/mL with assay medium (RPMI 1640 + 1% FBS), and 40 µL/well was added to the 96-well plate containing CHO-K1-PD-L1 cells. The plate was incubated in an incubator at 37°C with 5% CO₂ for 6 hours. After incubation, the 96-well plate was removed and equilibrated to room temperature. 80 µL of Bio-Glo detection reagent was added to each well, followed by incubation at room temperature for 5-10 minutes. The luminescence value was read using a Spectramax i3. All data were obtained from duplicate wells. The signal values were averaged, and the curves were fitted and plotted using the 4-parameter method.

The experimental results are shown in Figure 6B. The IC₅₀ values for the blockade of PD-1 and PD-L1 binding by anti-FGFR2/PD-1 bispecific antibody molecules a and b were 0.481 and 2.352 nM, respectively, while the IC₅₀ for the blockade of PD-1 and PD-L1 binding by the anti-PD-1 monoclonal antibody was 0.372 nM. This indicates that the ability of anti-FGFR2/PD-1 bispecific antibody molecule a to block PD-1 and PD-L1 binding is comparable to that of the anti-PD-1 monoclonal antibody, while the ability of anti-FGFR2/PD-1 bispecific antibody molecule b to block PD-1 and PD-L1 binding is reduced compared to the anti-PD-1 monoclonal antibody.

### Example 7. ADCC effect of anti-FGFR2/PD-1 bispecific antibodies on SNU-16 cells and T cells

This experiment used human gastric cancer cells SNU-16 with high surface expression of FGFR2 and Jurkat-PD-1-NFAT-luc cells expressing PD-1 as target cells to detect the killing ability of NK cells against tumor cells and T cells.

### 7.1 ADCC effect of bispecific antibodies on SNU-16 tumor cells

SNU-16 cells were harvested and resuspended to 2×10⁵ cells/mL with RPMI 1640 medium containing 5% FBS. 50 µL/well was added to the inner 60 wells of a 96-well plate, and the edge wells were sealed with sterile water. Anti-FGFR2/PD-1 bispecific antibodies a and b, and the positive control anti-FGFR2 monoclonal antibody were diluted to 200 nM with RPMI 1640 medium containing 5% FBS, followed by stepwise four-fold dilution, and then added to the wells containing cells at 50 µL/well. Incubated in a 37°C, 5% CO₂ incubator for 30 min. Meanwhile, fresh human peripheral blood mononuclear cells (hPBMC) were collected, and NK cells were isolated according to the instructions of the human NK Cell Isolation Kit (Miltenyi Biotec, #130-092-657). The isolated NK cells were resuspended to 4×10⁵ cells/mL with RPMI 1640 medium containing 5% FBS, and 100 µL/well was added to the above wells. The plate was then incubated in a 37°C, 5% CO₂ incubator for 24 h. LDH detection was performed according to the instructions of the CyQUANT^{™} LDH Cytotoxicity Assay Kit (Invitrogen, # C20300). Specifically, 20 µL of lysis buffer was added to the wells used to detect maximum LDH release, mixed gently by tapping, and incubated in a 37°C, 5% CO₂ incubator for 45 min. Thereafter, 50 µL of each sample was transferred to a new 96-well plate, and 50 µL of LDH detection working solution was added to each well. The plate was mixed thoroughly and incubated at room temperature, protected from light, for 30 minutes. Then, 50 µL of stop solution was added to each well, and the plate was mixed gently by tapping, and the OD values were measured at 490 nm and 680 nm using a microplate reader. Absorbance per well = OD₄₉₀ - OD₆₈₀. Cytotoxicity or mortality (%) = (Absorbance of experimental group - Absorbance of blank well) / (Absorbance of total LDH well - Absorbance of blank well) × 100.

The experimental results are shown in Figure 7A. Compared to the positive control anti-FGFR2 monoclonal antibody, the killing effect of NK cells on SNU-16 tumor cells was weaker under the action of anti-FGFR2/PD-1 bispecific antibody a; under the action of anti-FGFR2/PD-1 bispecific antibody b, NK cells exhibited significant killing effects on SNU-16 tumor cells. The EC₅₀ values for anti-FGFR2/PD-1 bispecific antibody b and the positive control anti-FGFR2 monoclonal antibody were 4.615 and 3.031 nM, respectively, indicating comparable levels of ADCC effect.

Therefore, after introducing the S239D-I332E point mutations into the Fc region of the anti-FGFR2/PD-1 bispecific antibody, the ADCC effect of anti-FGFR2/PD-1 bispecific antibody b was significantly enhanced (Figure 7A).

### 7.2 ADCC effect of bispecific antibodies on T cells

Jurkat-PD-1-NFAT-luc cells expressing PD-1 on the cell surface were used as target cells to detect the ADCC effect, following the method described above. The difference was that the antibodies used were anti-FGFR2/PD-1 bispecific antibodies a and b, the positive control anti-MHC-I monoclonal antibody, and the negative control anti-PD-1 monoclonal antibody. The initial antibody concentration was 50 nM, followed by stepwise four-fold dilution, and then added to the wells containing cells. LDH detection was performed according to the instructions of the CyQUANT^{™} LDH Cytotoxicity Assay Kit (Invitrogen, # C20300). Data analysis was performed using GraphPad Prism6.

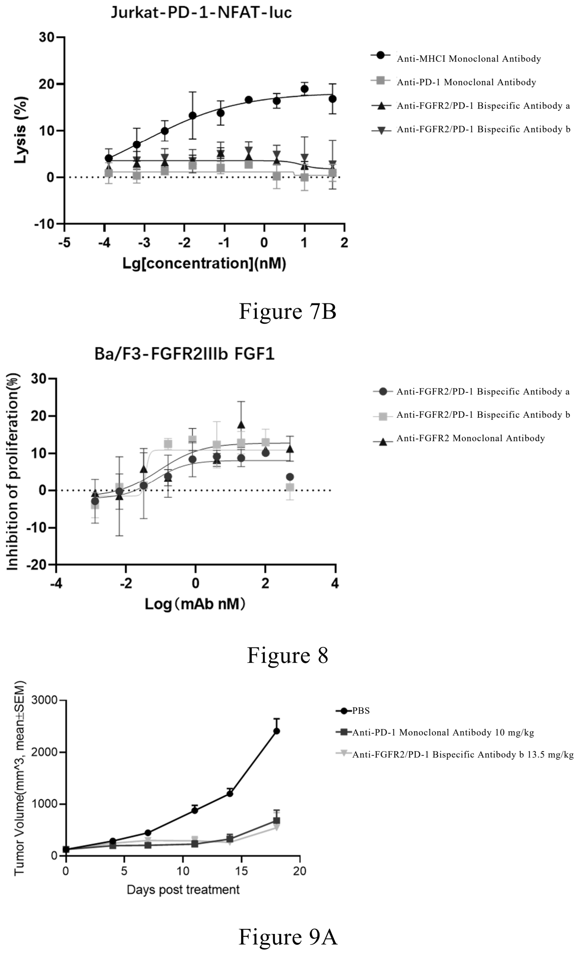

The results are shown in Figure 7B. NK cells exhibited significant killing effects under the action of the positive control anti-MHC-I monoclonal antibody, while under the action of anti-FGFR2/PD-1 bispecific antibodies a and b and the negative control anti-PD-1 monoclonal antibody, no significant killing effects on T cells were observed.

### Example 8. Inhibitory effect of anti-FGFR2/PD-1 bispecific antibodies on FGF1-induced proliferation of Ba/F3-FGFR2IIIb cells

After digestion with 0.25% trypsin-EDTA, 293FT cells were resuspended in DMEM complete medium and seeded into a 6-well plate at 700,000 cells per well. After cell adhesion, the viral packaging vector pLVX (containing the inserted human FGFR2IIIb gene) was transfected into 293FT cells using Lipofectamine 3000 transfection reagent. After 48 hours, the culture medium from 293FT cells was collected, centrifuged at 500g for 5 minutes, and the supernatant was collected. The supernatant was filtered through a 0.45 µm filter, and polybrene (a transduction enhancer) was added at a ratio of 1000:1. Then, 2 mL of the supernatant was added to a T25 culture flask (which had been seeded with 250,000 Ba/F3 cells 24 hours earlier). After 48 hours, Ba/F3 cells were collected and cultured in complete medium containing antibiotics. The cells were passaged until stable growth was achieved, resulting in the Ba/F3-FGFR2IIIb cell population. The Ba/F3-FGFR2 cell population was diluted with culture medium to 2-3 cells/mL and added to a 96-well plate for monoclonal sorting, yielding Ba/F3-FGFR2IIIb cell lines. The expression efficiency of the FGFR2IIIb gene in Ba/F3-FGFR2IIIb cell lines was detected by flow cytometry. The highest FGFR2IIIb gene expression efficiency in Ba/F3-FGFR2IIIb cell lines was 9.26% (results not shown).

Ba/F3-FGFR2IIIb cells cultured to the logarithmic phase were collected by centrifugation and diluted with complete medium to 1×10⁶ cells/mL. Then, 100 µL/well was added to a 3903 white clear-bottom 96-well plate and incubated at 37°C with 5% CO₂ for later use. Anti-FGFR2/PD-1 bispecific antibodies a and b, as well as anti-FGFR2 monoclonal antibody, were diluted to 500 nM with complete medium and subjected to stepwise fivefold dilution. The diluted solutions were added to the 96-well plate containing Ba/F3-FGFR2IIIb cells and incubated at 37°C with 5% CO₂ for 1 hour. FGF1 was diluted to a concentration of 10 ng/mL with complete medium, and heparin was diluted to a concentration of 5 µg/mL. These solutions were added to the 96-well plate containing Ba/F3-FGFR2IIIb cells and antibodies, followed by incubation at 37°C with 5% CO₂ for 2 hours. Then, 20 µL of MTS/PMS solution was added to each well, and the plate was incubated at 37°C with 5% CO₂ for 1-4 hours. After shaking and mixing, the OD value at 490 nm was measured using a microplate reader. All data were obtained from duplicate wells, and the signal values were converted to inhibition rates. The average values were used to plot curves using the 4-parameter fitting method.

The experimental results are shown in Figure 8. The EC₅₀ values for anti-FGFR2/PD-1 bispecific antibodies a and b and the positive control anti-FGFR2 monoclonal antibody were 0.0870, 0.0515, and 0.1030 nM, respectively. This indicates that anti-FGFR2/PD-1 bispecific antibody b has a stronger ability to compete with FGF1 for the FGFR2IIIb receptor, thereby better blocking FGF1-induced cell proliferation upon binding to FGFR2IIIb.

### Example 9. Anti-tumor effect of anti-FGFR2/PD-1 bispecific antibodies in the MC38 xenograft model

Mouse colon cancer MC38 cells cultured in vitro were collected, and the cell suspension concentration was adjusted to 2×10⁷ cells/mL. The right flank of NOG mice was shaved, and under sterile conditions, 100 µL of the cell suspension was subcutaneously inoculated into the right flank of NOG mice. After tumor cells formed solid tumors subcutaneously in the mice, the diameter of the xenografts was measured using a vernier caliper. When the average tumor volume reached 50 mm³-100 mm³, the animals were randomly divided into 3 groups, with 9 mice per group, including: a blank control group, injected with PBS only as a control; a control dose group of anti-PD-1 monoclonal antibody at 10 mg/kg; and a dose group of anti-FGFR2/PD-1 bispecific antibody b at 13.5 mg/kg. Administration was performed via intraperitoneal injection, twice a week, for a total of 5 doses. Throughout the experiment, the diameter of the xenografts was measured twice a week, and the body weight of the mice was recorded. Tumor growth curves over time and body weight curves for each group were plotted.

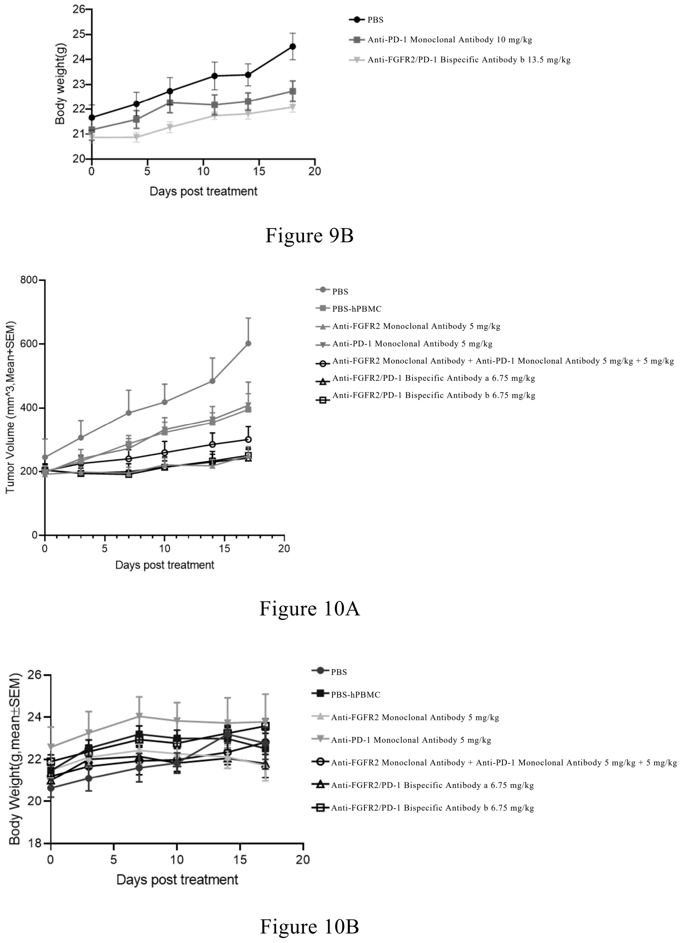

The experimental results are shown in Figures 9A and 9B. In the MC38 cell xenograft model, both anti-FGFR2/PD-1 bispecific antibody b and anti-PD-1 monoclonal antibody showed significantly greater tumor growth inhibition compared to the control group. There was no significant difference in tumor inhibition between anti-FGFR2/PD-1 bispecific antibody b and anti-PD-1 monoclonal antibody. Additionally, the body weight of mice in each dose group did not significantly decrease after several days of continuous administration, indicating that anti-FGFR2/PD-1 bispecific antibody b and anti-PD-1 monoclonal antibody had minimal toxic side effects on the mice.

The results indicate that in the xenograft model of non-target tumor cells, the anti-tumor activity of anti-FGFR2/PD-1 bispecific antibody b was not significantly different from that of anti-PD-1 monoclonal antibody. Both significantly inhibited tumor growth, demonstrating that the structure of the anti-FGFR2/PD-1 bispecific antibody does not interfere with the function of the anti-PD-1 monoclonal antibody.

### Example 10. Anti-tumor effect of anti-FGFR2/PD-1 bispecific antibodies in the SNU-16 xenograft model

Human gastric cancer SNU-16 cells cultured in vitro were collected, resuspended in serumfree medium, and mixed with an equal volume of Matrigel. The cell concentration was adjusted to 8×10⁷/mL. Under sterile conditions, 100 µL of the cell suspension was subcutaneously inoculated into the right flank of NOG mice. On day 8 after tumor inoculation, hPBMC cells were thawed, and the cell concentration was adjusted to 2.5×10⁷/mL. Except for 3 tumor-bearing mice that were not inoculated with hPBMC cells, the remaining tumor-bearing mice were injected with 200 µL of hPBMC cell suspension via the tail vein, corresponding to 5×10⁶ hPBMC cells per mouse. After tumor cells formed solid tumors subcutaneously in the mice, the diameter of the xenografts was measured using a vernier caliper. When the average tumor volume reached approximately 200 mm³, the animals were randomly divided into 7 groups, including: a blank control group-injected with PBS only as a control; an hPBMC control group-hPBMC cells diluted with PBS as a control; two single-antibody control groups-anti-FGFR2 monoclonal antibody at 5 mg/kg and anti-PD-1 monoclonal antibody at 5 mg/kg administered separately; one combination therapy control group-anti-FGFR2 monoclonal antibody at 5 mg/kg and anti-PD-1 monoclonal antibody at 5 mg/kg administered in combination; and two dose groups-anti-FGFR2/PD-1 bispecific antibody a at 6.75 mg/kg and anti-FGFR2/PD-1 bispecific antibody b at 6.75 mg/kg administered separately. Administration was performed twice a week, for a total of 5 doses. Throughout the experiment, the diameter of the xenografts was measured twice a week, and the body weight of the mice was recorded. Tumor growth curves over time and body weight curves for each group were plotted.

The experimental results are shown in Figures 10A, 10B, and Table 1. In the SNU-16 cell mixed hPBMC xenograft model, the tumor inhibitory effects of anti-FGFR2/PD-1 bispecific antibodies a and b showed no significant difference and were comparable to that of anti-FGFR2 monoclonal antibody. The inhibitory effects were relatively better compared to the combination of anti-FGFR2 monoclonal antibody and anti-PD-1 monoclonal antibody. The tumor inhibitory effects of anti-FGFR2/PD-1 bispecific antibodies a and b were significantly superior to that of anti-PD-1 monoclonal antibody. Additionally, the body weight of the mice did not significantly decrease after several days of continuous administration, indicating that anti-FGFR2/PD-1 bispecific antibodies a and b had minimal toxic side effects on the mice.

The results indicate that in the SNU-16 cell xenograft model with high FGFR2 expression, the tumor inhibitory activities of anti-FGFR2/PD-1 bispecific antibodies a and b were superior to that of anti-PD-1 monoclonal antibody. Both significantly inhibited tumor growth, demonstrating that anti-FGFR2/PD-1 bispecific antibodies can specifically target FGFR2-expressing tumor cells and exert favorable anti-tumor effects.

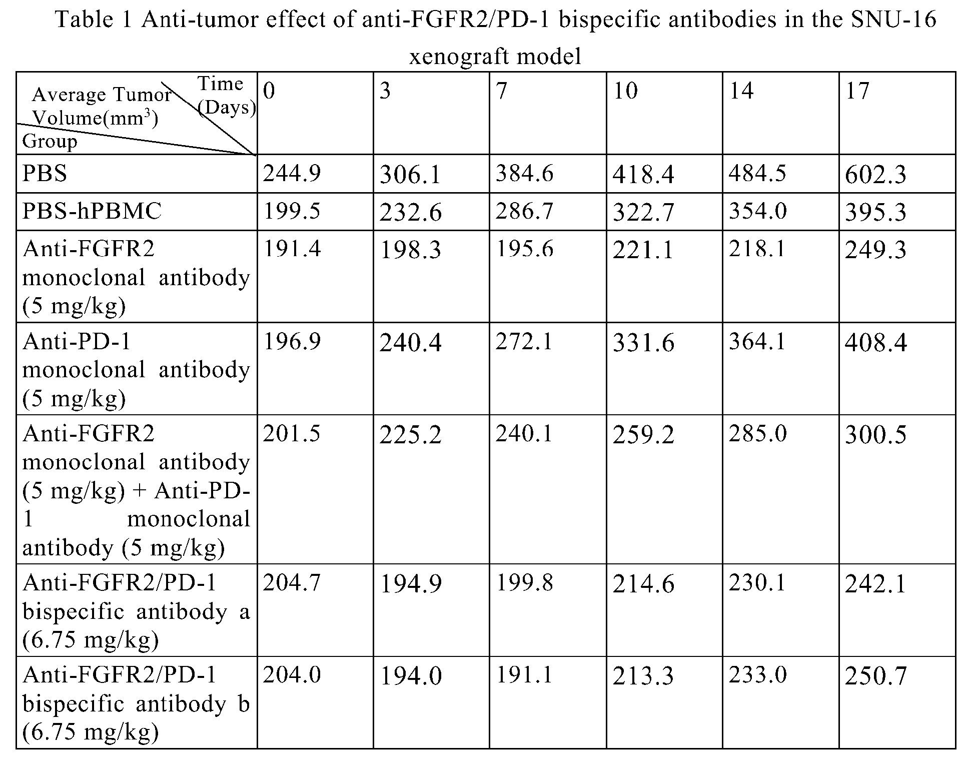

### Example 11. Stability study of anti-FGFR2/PD-1 bispecific antibody b

This experiment can be used to evaluate the stability of the protein in the presence of excipients, thereby revealing important information required for the development of an optimal formulation.

Anti-FGFR2/PD-1 bispecific antibody b was dialyzed overnight in Buffer 1 (20 mM acetate + 6% trehalose + 1% arginine hydrochloride + 0.2% Tween 80, pH 5.0) and Buffer 2 (20 mM histidine + 6% trehalose + 50 mM sodium chloride + 0.2% Tween 80, pH 5.5), followed by ultrafiltration concentration at 4000 rpm for 20 minutes to 5 mg/mL. The samples were aliquoted and stored at 4°C, 25°C, and 37°C, respectively.

The experimental results are shown in Table 2. Under the same treatment conditions in different buffers, significant aggregates appeared in anti-FGFR2/PD-1 bispecific antibody b in Buffer 1 on day 21, but no aggregates were observed in Buffer 2 until day 28, when a small amount of aggregates was detected. This indicates that anti-FGFR2/PD-1 bispecific antibody b exhibits better stability in the acetate-based buffer.

**Table 2 Preliminary screening results of anti-FGFR2/PD-1 bispecific antibody formulations**

| **Histidine-Buffer (pH 5.5)** | | | | | **Acetate-Buffer (pH 5.0)** | | | | |
|---|---|---|---|---|---|---|---|---|---|
| Time (Days) | 25°C | | 37°C | | Time (Days) | 25°C | | 37°C | |
| | Aggregate (%) | Purity (%) | Aggregate (%) | Purity (%) | | Aggregate (%) | Purity (%) | Aggregate (%) | Purity (%) |
| 0 | 1.46 | 98.54 | 1.46 | 98.54 | 0 | 1.46 | 98.54 | 1.46 | 98.54 |
| 7 | 0 | 100 | 0 | 100 | 7 | 0 | 100 | 0 | 100 |
| 14 | 0 | 100 | 0 | 100 | 14 | 0 | 100 | 0 | 100 |
| 21 | 2.66 | 97.34 | 1.9 | 98.1 | 21 | 0 | 100 | 0 | 100 |
| 28 | \ | \ | \ | \ | 28 | 0.16 | 99.84 | 0.36 | 99.64 |

### Discussion

From the above experiments, it is evident that the bispecific antibody provided by the present invention can simultaneously bind to FGFR2 and PD-1, releasing the immune activity function of T cells by binding to PD-1, and specifically killing tumor cells expressing the FGFR2 protein. Anti-FGFR2/PD-1 bispecific antibodies can effectively block the binding of PD-1 and PD-L1. Among them, bispecific antibody b exhibited significant proliferation inhibition on target cells SNU-16 and relatively better inhibition of cell proliferation by competing with FGF1 for the FGFR2 receptor. After introducing the S239D-I332E point mutations into the Fc region of anti-FGFR2/PD-1 bispecific antibodies, the ADCC effect of anti-FGFR2/PD-1 bispecific antibody b was significantly enhanced (Figure 7A), but no significant killing effect on T cells was observed. In xenograft models, whether in non-target protein MC38 cells or FGFR2-high-expressing SNU-16 cells, anti-FGFR2/PD-1 bispecific antibodies demonstrated favorable anti-tumor activity.

During the study of anti-FGFR2/PD-1 bispecific antibodies, anti-FGFR3/PD-1 bispecific antibodies were also designed. However, in multiple animal model efficacy studies, it was found that the anti-tumor activity of anti-FGFR3/PD-1 bispecific antibodies was significantly weaker than that of anti-FGFR2/PD-1 bispecific antibodies and anti-PD-1 monoclonal antibody. This indicates that the combination of anti-FGFR3 monoclonal antibody and anti-PD-1 monoclonal antibody may interfere with the activity of anti-PD-1 monoclonal antibody, further validating the superior efficacy of the two-target combination in the anti-FGFR2/PD-1 bispecific antibody of the present invention. Without affecting the release of T cell immune activity function, the antibody effect on target cells is also favorable, enabling specific binding and killing of FGFR2-expressing tumor cells.

All publications mentioned in the present invention are incorporated in the present application by reference to the same extent as if each individual publication was individually indicated to be incorporated by reference. Furthermore, it should be understood that, after reading the above teachings of the present invention, those skilled in the art can make various changes or modifications to the present invention, and these equivalent forms shall also fall within the scope defined by the claims attached to the present application.

## Claims

1. A bispecific antibody, comprising:
a first antigen-binding domain D1; and
a second antigen-binding domain D2,
wherein, said D1 specifically binds to the target molecule FGFR2 protein,
said D2 specifically binds to the target molecule PD-1 protein;
wherein, D1 is an anti-FGFR2 antibody or an antigen-binding fragment thereof, said anti-FGFR2 antibody or antigen-binding fragment thereof comprises a heavy chain variable region and a light chain variable region, said heavy chain variable region comprises the following three heavy chain complementarity determining regions: HCDR1 as shown in SEQ ID NO. 1, HCDR2 as shown in SEQ ID NO. 2, HCDR3 as shown in SEQ ID NO. 3; and
said light chain variable region comprises the following three light chain complementarity determining regions: LCDR1 as shown in SEQ ID NO. 4, LCDR2 as shown in SEQ ID NO. 5, LCDR3 as shown in SEQ ID NO. 6.

2. The bispecific antibody according to claim 1, **characterized in that** said D2 is an anti-PD-1 antibody or an antigen-binding fragment thereof; said anti-PD-1 antibody or antigen-binding fragment thereof comprises a heavy chain variable region and a light chain variable region, said heavy chain variable region comprises the following three heavy chain complementarity determining regions: HCDR1 as shown in SEQ ID NO. 7, HCDR2 as shown in SEQ ID NO. 8, HCDR3 as shown in SEQ ID NO. 9; and
said light chain variable region comprises the following three light chain complementarity determining regions: LCDR1 as shown in SEQ ID NO. 10, LCDR2 as shown in SEQ ID NO. 11, LCDR3 as shown in SEQ ID NO. 12.

3. The bispecific antibody according to claim 1, **characterized in that** said anti-FGFR2 or anti-PD-1 antigen-binding fragment is selected from the group consisting of Fab, F(ab'), F(ab')₂, Fv or single-chain Fv (scFv); said anti-FGFR2 or anti-PD-1 antibody is an IgG antibody.

4. The bispecific antibody according to claim 1, **characterized in that** D1 is an anti-FGFR2 antibody, and D2 is connected via a linker to a region of D1 selected from the group consisting of: the heavy chain variable region, the heavy chain constant region, the light chain variable region, or a combination thereof.

5. The bispecific antibody according to claim 1, **characterized in that** said bispecific antibody comprises a monomer, having a structure represented by Formula I or Formula II from the N-terminus to the C-terminus: 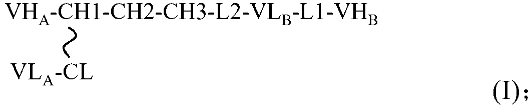 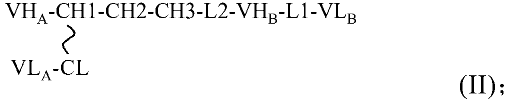 wherein,
VL_{A} represents the light chain variable region of the anti-FGFR2 antibody or antigen-binding fragment thereof;
VH_{A} represents the heavy chain variable region of the anti-FGFR2 antibody or antigen-binding fragment thereof;
VL_{B} represents the light chain variable region of the anti-PD-1 antibody or antigen-binding fragment thereof;
VH_{B} represents the heavy chain variable region of the anti-PD-1 antibody or antigen-binding fragment thereof;
CH represents the heavy chain constant region;
CL represents the light chain constant region;
L1 and L2 are each independently a bond or a linker;
"~" represents a disulfide bond or covalent bond;
"-" represents a peptide bond;
wherein, said bispecific antibody has the activity of simultaneously binding FGFR2 and binding PD-1.

6. The bispecific antibody according to claim 1, **characterized in that** said anti-FGFR antibody has a heavy chain constant region comprising an amino acid mutation selected from the group consisting of:
a) the heavy chain constant region has an amino acid mutation at position 239; preferably S293D; and/or
b) the heavy chain constant region has an amino acid mutation at position 332; preferably I332E.

7. The bispecific antibody according to claim 1, **characterized in that** said anti-FGFR2 antibody or antigen-binding fragment thereof comprises a heavy chain variable region and a light chain variable region, wherein the heavy chain variable region amino acid sequence is as shown in SEQ ID NO. 13, and the light chain variable region amino acid sequence is as shown in SEQ ID NO. 14; and/or, said anti-PD1 antibody or antigen-binding fragment thereof comprises a heavy chain variable region and a light chain variable region, wherein the heavy chain variable region amino acid sequence is as shown in SEQ ID NO. 15, and the light chain variable region amino acid sequence is as shown in SEQ ID NO. 16.

8. The bispecific antibody according to claim 1, **characterized in that** the heavy chain constant region sequence of said anti-FGFR antibody is as shown in SEQ ID NO. 23; or the heavy chain constant region sequence of said anti-FGFR antibody is as shown in SEQ ID NO. 24.

9. The bispecific antibody according to claim 1, **characterized in that** said bispecific antibody comprises a heavy chain and a light chain, and the amino acid sequences of said heavy chain and light chain are selected from the group consisting of:
a) the heavy chain amino acid sequence of said bispecific antibody is as shown in SEQ ID NO. 29, and the light chain amino acid sequence of said bispecific antibody is as shown in SEQ ID NO. 28;
b) the heavy chain amino acid sequence of said bispecific antibody is as shown in SEQ ID NO. 30, and the light chain amino acid sequence of said bispecific antibody is as shown in SEQ ID NO. 28; or,
c) a polypeptide derived from a), b) or c) formed by substitution, deletion or addition of one or more amino acid residues to the amino acid sequence in a) or b), and having both anti-FGFR activity and anti-PD1 activity.

10. A polynucleotide molecule, **characterized in that** said polynucleotide molecule encodes the bispecific antibody according to any one of claims 1-9.

11. An expression vector, **characterized in that** said expression vector contains the polynucleotide molecule according to claim 10.

12. A host cell, **characterized in that** said host cell contains the expression vector according to claim 11.

13. A method for preparing the bispecific antibody according to any one of claims 1-9, **characterized in that** said preparation method comprises the following steps:
a) culturing the host cell according to claim 12 under expression conditions, thereby expressing the bispecific antibody;
b) isolating and purifying the bispecific antibody from step a).

14. A pharmaceutical composition, **characterized in that** said pharmaceutical composition comprises an effective amount of the bispecific antibody according to any one of claims 1-9 and one or more pharmaceutically acceptable carriers, diluents or excipients.

15. Use of the bispecific antibody according to any one of claims 1-9 or the pharmaceutical composition according to claim 14 in the manufacture of a drug for cancer or tumor; preferably, said cancer or tumor is an FGFR2-positive cancer or tumor.

16. An immunoconjugate, **characterized in that** said immunoconjugate comprises:
a) the bispecific antibody according to any one of claims 1-9; and
a conjugation moiety selected from the group consisting of: a detectable label, a drug, a toxin, a cytokine, a radionuclide, or an enzyme.
